(19) Europäisches Patentamt

European Patent Office

Office européen des brevets

(11) **EP 1 221 297 B1**

(12) **EUROPÄISCHE PATENTSCHRIFT**

(45) Veröffentlichungstag und Bekanntmachung des Hinweises auf die Patenterteilung:
**10.09.2003 Patentblatt 2003/37**

(51) Int Cl.[7]: **A61B 5/00**, G06F 17/13

(21) Anmeldenummer: **00128714.3**

(22) Anmeldetag: **29.12.2000**

(54) **Verfahren zur Todeszeitpunktbestimmung**

Method for determination of the time of death

Méthode pour déterminer le temps de la mort

(84) Benannte Vertragsstaaten:
**AT CH DE LI**

(43) Veröffentlichungstag der Anmeldung:
**10.07.2002 Patentblatt 2002/28**

(73) Patentinhaber: **Mall, Else-Gita, Dr.**
**82377 Penzberg (DE)**

(72) Erfinder: **Mall, Else-Gita, Dr.**
**82377 Penzberg (DE)**

(74) Vertreter: **Weigel, Matthias, Dipl.-Ing. et al**
**Meissner, Bolte & Partner**
**Postfach 86 03 29**
**81630 München (DE)**

(56) Entgegenhaltungen:
- **M.A.GREEN, J.C.WRIGHT: "Postmortem Interval Estimation from Body Temperature Data only" FORENSIC SCIENCE INTERNATIONAL, Bd. 28, 1985, Seite 35-46 XP001000857**
- **K. HIRAIWA ET AL.: "estimation of Postmortem Interval from Rectal Temperature by Use of Computer" MED. SCI. LAW, Bd. 20, Nr. 2, 1980, XP001000885**
- **DATABASE COMPENDEX [Online] ENGINEERING INFORMATION, INC., NEW YORK, NY, US; HAYES L J ET AL: "FINITE ELEMENT MODEL FOR THE EXPOSURE OF A COMPOSITE MAN WITH DISTRIBUTED INTERNAL HEAT GENERATION TO A CONVECTIVE SUBFREEZING ENVIRONMENT"** Database accession no. EIX82060007507 XP002170817 & NOV 15-20, 1981, ASME Pap 1981
- **DATABASE COMPENDEX [Online] ENGINEERING INFORMATION, INC., NEW YORK, NY, US; CAO ZUO ET AL: "Finite element analysis of human thermal regulating system"** Database accession no. EIX99384738681 XP002170818 & BEIJING, CHINA, Bd. 23, Nr. 5, 1997, Seiten 551-554,

EP 1 221 297 B1

**Beschreibung**

Die Erfindung betrifft ein Verfahren zur individuellen Bestimmung des Todeszeitpunktes eines biologischen Organismus, insbesondere eines Menschen, wobei bei dem Verfahren eine von einem Anfangstemperaturniveau ausgehende, theoretische zeitliche Temperaturänderung des Organismus vorgegeben wird, deren Anfangszeitpunkt einen theoretischen Todeszeitpunkt des Organismus definiert, und mindestens ein am Organismus nach dessen Ableben gemessener Temperaturwert mit der theoretischen Temperaturänderung verglichen wird, wobei der dem gemessenen Temperaturwert in der theoretischen Temperaturänderung zugeordnete theoretische Messzeitpunkt als tatsächlicher Messzeitpunkt der Temperaturmessung und der sich aus dem theoretischen Messzeitpunkt ergebende theoretische Todeszeitpunkt als tatsächlicher Todeszeitpunkt definiert wird.

Die Bestimmung des Todeszeitpunktes beim Menschen spielt in der rechtsmedizinischen Praxis eine wichtige Rolle. Eine mehr oder weniger exakte Bestimmung des Todeszeitpunktes gelingt jedoch nur in der frühen postmortalen Phase, d.h. innerhalb eines Zeitraums von bis zu einigen Tagen nach dem Tod.

Da die exakte Todeszeitpunktbestimmung als Bestimmung des mutmaßlichen Tatzeitpunktes insbesondere bei der juristischen Bewertung von Tötungsdelikten beispielsweise zur Überprüfung von Alibis eine überragende Bedeutung erlangt hat, wurden bereits Ende des 19. Jahrhunderts Verfahren zur Bestimmung des Todeszeitpunktes diskutiert und entwickelt. Dabei hat sich herausgestellt, dass temperaturorientierte Verfahren zur Todeszeitpunktbestimmung, die auf einer zeitlichen Temperaturänderung der Leiche basieren, die genauesten Rückschlüsse über den tatsächlichen Todeszeitpunkt ermöglichen.

Aus dem Stand der Technik sind verschiedene Ansätze und Modelle zur Bestimmung des Todeszeitpunktes bekannt.

So sind aus der rechtsmedizinischen Literatur verschiedene physikalische Modelle bekannt, bei denen der Körper des Menschen durch einen homogenen Zylinder unendlicher Länge ersetzt wird, an dem die Wärmetransfermechanismen, Konduktion, Konvektion und Strahlung, auf vereinfachte Weise beobachtet und berechnet werden können. Ein derartiges Modell wird von Sellier (Determination of the time of death by extrapolation of the temperature decrease curve, Acta Medicinae Socialis et Legalis 11: 279 - 302, 1958), von Joseph und Schickele (A general method for assessing factors controlling postmortem cooling, Journal of Forensic Sciences 15 (3): 364 - 391, 1970) und von Hiraiwa et al. beschrieben (Estimation of postmortem interval from rectal temperature by use of Computer, Medicine Science and Law 20 (2): 115 - 125, 1980 und Estimation of postmortem interval from rectal temperature by use of computer - Relationship between the rectal and skin cooling curves, Medicine Science and Law 21 (1): 4 - 9, 1981).

Zu diesen sogenannten physikalischen Modellen wurden alternativ mathematische Modelle entwickelt, bei denen auf Grundlage tatsächlicher Messungen an Verstorbenen empirische Abkühlkurven aufgestellt und auf deren Grundlagen mathematische Modelle zur Berechnung des Todeszeitpunktes entwickelt wurden. Ein derartiges mathematisches Modell wird von DeSaram et al. (Post-mortem temperature and the time of death, Journal of Criminal Law, Criminology and Police Science 46: 562 - 577, 1955) von Fiddes and Patten (A percentage method for representing the fall in body temperature after death, Journal of Forensic Medicine 5 (1): 2 - 15, 1958), von Marshall and Hoare (Estimating the time of death - The rectal cooling after death and its mathematical expression, Journal of Forensic Sciences 7 (1): 56 - 81, Estimating the time of death - The use of the cooling formula in the study of postmortem body cooling, Journal of Forensic Sciences 7 (2): 189 - 210 und Estimating the time of death - the use of body temperature in estimating the time of death, Journal of Forensic Sciences 7 (2): 211-221.) oder von Green and Wright (The theoretical aspects of the time dependent Z equation as a means of postmortem interval estimation using body temperature data only, Forensic Science International 28: 53 - 62, 1985 und Postmortem interval estimation from body temperature data only, Forensic Science International 28: 35 - 46, 1985) beschrieben.

Das derzeit auch international nahezu ausschließlich verwendete und vor Gericht vertretene Verfahren basiert auf dem doppelt - exponentiellen Modell nach Marshall and Hoare unter Verwendung eines von Henßge et al. entwickelten Nomogramms, wobei gleichzeitig eine Parameterfestlegung nach Henßge durchgeführt wird (Methoden zur Bestimmung der Todeszeit an Leichen, Schmid-Römhild, Lübeck, 1988, Die Präzision von Todeszeitschätzungen durch die mathematische Beschreibung der rektalen Leichenabkühlung, Zeitschrift für Rechtsmedizin 83: 49 - 67, 1979 sowie Todeszeitschätzungen durch die mathematische Beschreibung der rektalen Leichenabkühlung unter verschiedenen Abkühlbedingungen, Zeitschrift für Rechtsmedizin 87: 147 - 178, 1981). Bei diesem derzeit gängigen Verfahren werden basierend auf der Formel nach Marshall and Hoare die folgenden Gesetzmäßigkeiten aufgestellt:

$$T_u \leq 23{,}2°C$$

$$T_{r1} = ((37{,}2 - T_u)\,1{,}25\,(e^{-Zt}) - (37{,}2 - T_u)\,0{,}25\,(e^{-5Zt})) + T_u$$

$$T_u \geq 23{,}3 \; ^\circ C$$

$$T_{r1} = ((37{,}2 - T_u) \, 1{,}11 \, (e^{-Zt}) - (37{,}2 - T_u) \, 0{,}11 \, (e^{-10Zt})) + T_u$$

mit

$$Z = 0{,}0284 \text{ bis } 1{,}2815 \, [kg]^{-0{,}625}$$

dabei entspricht $T_u$ der Umgebungstemperatur, $T_{r1}$ der gemessenen Rektaltemperatur, t der Zeit seit dem Todeseintritt sowie Z einem körpergewichtabhängigen Parameter. Dieser vom Körpergewicht abhängige Parameter Z kann durch entsprechende sogenannte Körpergewichtskorrekturfaktoren, mit denen unterschiedliche Randbedingungen am Leichenfundort berücksichtigt werden können, wie beispielsweise die Feuchtigkeit und Luftbewegung (Körpergewichtskorrekturfaktor <1), die Bekleidung oder die Bedeckung (Körpergewichtskorrekturfaktor >1), angepaßt werden.

Bei diesem bekannten Verfahren von Henßge wird die Rektaltemperatur der Leiche am Fundort gemessen und anschließend auf Grundlage der gemessenen Rektaltemperatur die Länge des Zeitintervalls zwischen Todeseintritt und Messzeitpunkt durch eine Inversion der Formel bestimmt.

Dieses Verfahren nach Henßge ist nur in solchen Fällen anwendbar, in denen die Leiche bis zur Temperaturmessung bei konstanten Umgebungsbedingungen am Fundort lag. Unter Standardbedingungen ist dann eine sichere Bestimmung des todeszeitpunktes mit einem Fehler von +/- 2,8 Stunden, unter sogenannten Nicht-Standard-Bedingungen nur in einem Intervall von bis zu vierzehn Stunden möglich, so dass eine insbesondere für die juristische Bewertung nicht ausreichende Verlässlichkeit dieses Verfahrens gegeben ist. Bestimmte Nicht-Standard-Bedingungen wie z.B. eine externe Einstrahlung oder sich ändernde Umgebungsbedingungen können mit dem bekannten Verfahren nicht beurteilt werden.

Es ist daher Aufgabe der Erfindung, ein Verfahren zur individuellen Bestimmung des Todeszeitpunktes eines biologischen Organismus, insbesondere eines Menschen, anzugeben, mit dessen Hilfe ohne großen Aufwand eine, verglichen mit den bekannten Verfahren genauere Todeszeitpunktbestimmung des Organismus möglich ist.

Die Erfindung löst die Aufgabe durch ein Verfahren mit den Merkmalen nach Anspruch 1 und insbesondere dadurch, dass ein Modellkörper des Organismus aus einer Vielzahl von Volumenelementen generiert wird, dass zumindest einem Teil der Volumenelemente jeweils wenigstens eine definierte thermodynamische Eigenschaft des Organismus zugeordnet wird, dass zumindest teilweise der Einzelwärmetransfer zwischen benachbart zueinander angeordneten Volumenelementen ermittelt wird, dass basierend auf den thermodynamischen Eigenschaften der Volumenelemente und basierend zumindest auf einem Teil der ermittelten Einzelwärmetransfers zwischen den Volumenelementen als Gesamtwärmetransfer die Wärmeaufnahme und die Wärmeabgabe des Modellkörpers bezogen auf die Zeit bestimmt wird und dass auf Grundlage des Gesamtwärmetransfers die theoretische zeitliche Temperaturänderung des Organismus bestimmt wird.

Ein wesentlicher Gedanke der Erfindung beruht darauf, den Organismus, dessen Todeszeitpunkt bestimmt werden soll, als Modellkörper aus einer Vielzahl von Volumenelementen zu generieren, wobei zumindest einem Teil der Volumenelemente jeweils wenigstens eine definierte thermodynamische Eigenschaft des Organismus zugeordnet wird. Auf diese Weise wird ein Modell generiert, das bei entsprechend geeigneter Wahl der thermodynamischen Eigenschaften, wie beispielsweise der spezifischen Wärmekapazität, der Wärmeleitfähigkeit oder der Massendichte des Organismus, zumindest annähernd ein Temperaturverhalten beim Abkühlen zeigt, dass mit dem tatsächlichen Abkühlverhalten des Organismus vergleichbar ist.

Um den Wärmetransport im Modellkörper zu simulieren, werden zumindest teilweise die Einzelwärmetransfers zwischen benachbart zueinander angeordneten Volumenelementen ermittelt, so dass der Modellkörper bei Vorgabe entsprechender Anfangs- und Randbedingungen ein Abkühlverhalten zeigt, von dem angenommen werden kann, dass es zumindest annähernd dem tatsächlichen Abkühlverhalten des Organismus bei entsprechenden Anfangs- und Randbedingungen entspricht.

Ein weiterer Gedanke der Erfindung liegt darin, basierend auf diesen vorgegebenen thermodynamischen Eigenschaften der Volumenelemente und basierend zumindest auf einem Teil der Einzelwärmetransfers die Wärmeaufnahme und die Wärmeabgabe des Modellkörpers entsprechend den vorgegebenen Anfangs- und Randbedingungen zu bestimmen. Auf Grundlage dieser als Gesamtwärmetransfer bezeichneten Wärmeänderung des Modellkörpers kann eine theoretische zeitliche Temperaturänderung an jedem Ort des Organismus bestimmt werden, mit deren Hilfe anschließend in bekannter Weise der tatsächliche Todeszeitpunkt bestimmt werden kann.

Zur Bestimmung des Gesamtwärmetransfers auf Grundlage der thermodynamischen Eigenschaften der Volumenelemente und der Einzelwärmetransfers zwischen den Volumenelementen können verschiedene, bekannte numerische Verfahren eingesetzt werden. Hierzu wird auf Grundlage der vorgegebenen thermodynamischen Eigenschaften sowie auf Grundlage vorgegebener Anfang- und Randbedingungen, wie dem durch den Modellkörper vorgegebenen Volumen, dem Anfangstemperaturniveau bzw. Anfangstemperaturfeld oder der Umgebungstemperatur, die partielle Wärmeleitungsgleichung, die den Gesamtwärmetransfer im Organismus beschreibt, numerisch gelöst. Auf Grundlage dieser numerischen Lösung werden die theoretischen Temperaturänderungen an jedem Ort des Organismus über die Zeit berechnet.

Die Volumenelemente sind üblicherweise polyederförmig und werden normalerweise als gegebenenfalls verzerrte Würfel oder Quader ausgebildet, wodurch einerseits eine vergleichsweise genaue Nachahmung der Form des Organismus als Modellkörper möglich ist und gleichzeitig der Gesamtwärmetransfer mit im Vergleich zur Anzahl der Volumenelemente geringem Aufwand bestimmt werden kann.

Durch das erfindungsgemäße Verfahren ist eine verglichen mit dem bekannten Verfahren genauere und gleichzeitig auch umfassende (d.h. für alle denkbaren Umgebungsbedingungen gültige) Todeszeitpunktbestimmung des Organismus möglich.

Weitere vorteilhafte Weiterbildungen des erfindungsgemäßen Verfahrens ergeben sich aus den Unteransprüchen.

So ist es besonders vorteilhaft, wenn zusätzlich zum Modellkörper zumindst teilweise auch eine unmittelbare Modellumgebung des Modellkörpers aus Volumenelementen generiert wird. Zumindest einem Teil dieser Volumenelemente der Modellumgebung wird wenigstens eine definierte thermodynamische Eigenschaft der tatsächlichen Umgebung zugeordnet, in der der Organismus aufgefunden worden ist, beispielsweise dem Fundort einer Leiche. Bei der Bestimmung des Gesamtwärmetransfers werden die thermodynamischen Eigenschaften der Volumenelemente des Modellkörpers und die der Volumenelemente der Modellumgebung sowie auch die Einzelwärmetransfers zwischen den Volumenelementen der Modellumgebung und des Modellkörpers mitberücksichtigt. Auf diese Weise ist es möglich, auch sich ändernde Umgebungsbedingungen bei der Bestimmung des Gesamtwärmetransfers mit zu berücksichtigen. Darüber hinaus ist es möglich, am Modellkörper beispielsweise Kleidungsstücke mit entsprechenden thermodynamischen Eigenschaften als Modellumgebung aus Volumenelementen zu generieren.

Den Volumenelementen des Modellkörpers werden als thermodynamische Eigenschaften die thermische Konduktivität, die spezifische Wärmekapazität und/oder die Massendichte, zumindest eines Gewebetyps des Gewebes des Organismus zugeordnet. Den Volumenelementen der Modellumgebung werden, sofern diese vorgesehen sind, entsprechende thermische Eigenschaften zugeordnet.

Zusätzlich zu zumindest einer der zuvor genannten thermischen Eigenschaften wird den die Oberfläche des Modellkörpers begrenzenden Volumenelementen bei einer bevorzugten Ausführungsform des Verfahrens zusätzlich als thermodynamische Eigenschaft die Emissivität zugeordnet. Auf diese Weise ist es möglich, die Wärmeabstrahlung und die Absorption externer Strahlung des Modellkörpers an der Oberfläche zu beschreiben.

Um eine möglichst exakte Darstellung des theoretischen Gesamtwärmetransfers des Modellkörpers zu erreichen, wird ferner vorgeschlagen, den Modellkörper in mehrere Körperkompartimente definierende Gruppen von Volumenelementen zu untergliedern, wobei jeder Gruppe von Volumenelementen mindestens eine definierte thermodynamische Eigenschaft eines bestimmten Gewebetyps des Organismus zugeordnet wird. So wird beispielsweise vorgeschlagen, bei der Generierung eines Menschen als Modellkörper beispielsweise die Haut, das Fettgewebe, das Skelettsystem, die Muskulatur, oder die inneren Organe jeweils als Gruppen von Volumenelementen zusammenzufassen, wobei jeder Gruppe von Volumenelementen, die für den jeweiligen Gewebetyp spezifischen thermodynamischen Eigenschaften zugeordnet werden. Auf diese Weise ist es möglich, beispielsweise die verglichen mit der anderer Gewebearten geringe Wärmeleitfähigkeit von Fettgewebe bei der Bestimmung des Gesamtwärmetransfers mitzuberücksichtigen. Bei bestimmten faserigen Gewebetypen, wie beispielsweise dem Muskelgewebe mit einer dominierenden Faservorzugsrichtung, wird darüber hinaus vorgeschlagen, die Anisotropie in der Wärmeleitfähigkeit derartiger Gewebetypen bei der Bestimmung des Gesamtwärmetransfers gleichfalls zu berücksichtigen.

Bei einer besonders bevorzugten Ausführungsform des erfindungsgemäßen Verfahrens wird ferner vorgeschlagen, bei der Bestimmung des Gesamtwärmetransfers die am Modellkörper auftretende Konvektion, Konduktion und/oder Strahlung mit einzubeziehen. Auf diese Weise ist es möglich, am Modellkörper auftretende Wasser- oder Luftströmungen oder auch auf den Modellkörper einwirkende Wärmestrahlung zu simulieren, wodurch beispielsweise die an einem Leichenfundort tatsächlich herrschenden Umgebungsbedingungen, wie Wind, Wasser und Sonnen- oder Heizungseinstrahlung, bei der Bestimmung des Gesamtwärmetransfers mit einbezogen werden können.

Darüber hinaus wird bei dem erfindungsgemäßen Verfahren vorgeschlagen, bei der Bestimmung des Gesamtwärmetransfers eine postmortale Wärmeproduktion des Organismus mitzuberücksichtigen, wobei die postmortale Wärmeproduktion ausgehend von derjenigen zum theoretischen Todeszeitpunkt linear oder exponentiell abnimmt. So hat sich bei medizinischen Untersuchungen gezeigt; dass unmittelbar nach dem Tod eines Menschen ein Großteil der Zellen nach wie vor aktiv ist und die noch im Körper verfügbaren Nährstoffe zumindest teilweise verarbeitet. Da durch die fehlende Blutzirkulation ein Abtransport der Wärme im Körper nicht mehr gegeben ist, kann es durch diese postmortale Wärmeproduktion zu einer Wärmeentwicklung innerhalb des Leichnams kommen, die so hoch ist, dass der Leichnam erst verzögert abkühlt. Diese postmortale Wärmeproduktion wird bei dem Verfahren bei der Bestimmung des Gesamtwärmetransfers mitberücksichtigt, wobei es zusätzlich möglich ist, beispielsweise bei schweren Infektionen des zu begutachtenden Organismus, eine postmortale Wärmeproduktion zu erfassen, die sogar zu einer Erhöhung der Kerntemperatur des Modellkörpers führt, um so eine postmortale Temperaturerhöhung am Modellkörper zu simulieren.

Als Basis für die innere postmortale Wärmeproduktion des Organismus wird der theoretische Grundumsatz des betreffenden Organismus unmittelbar nach dessen Tod herangezogen. Der Grundumsatz kann gegebenenfalls auch in Abhängigkeit vom Alter, vom Gewicht oder auch vom Allgemeinzustand des Organismus variieren und entsprechend am Modellkörper verändert werden.

Um einen möglichst natürlichen Abfall der postmortalen Wärmeproduktion zu simulieren, wird bei dem erfindungsgemäßen Verfahren ferner vorgeschlagen, den postmortalen zeitlichen Verlauf der inneren Leistung durch einen exponentiellen Abfall darzustellen, wobei die Abfallrate der inneren Leistung vorzugsweise auf einen Wert gesetzt wird, der einer Halbwertszeit von etwa zwei bis drei Stunden der inneren Wärmeleistung entspricht.

Um auch den tatsächlichen Entstehungsort der inneren Wärmeproduktion am Modellkörper mitzuberücksichtigen, wird bei einer bevorzugten Ausführungsform des erfindungsgemäßen Verfahrens ferner vorgeschlagen, die innere Wärmeproduktion prozentual auf mehrere Körperkompartimente des Organismus bildende Gruppen von Volumenelementen unterschiedlich zu verteilen. So werden beispielsweise, wenn das erfindunsgemäße Verfahren zur Bestimmung des Todeszeitpunktes eines Menschen herangezogen wird, bei der Generierung des Modellkörpers 55 bis 65 % des Grundumsatzes auf den Rumpfkern, 16 bis 20 % des Grundumsatzes auf die Muskulatur, 15 bis 19 % des Grundumsatzes auf das Gehirn und 4 bis 6 % des Grundumsatzes auf die Knochen und das Bindegewebe verteilt.

Des Weiteren wird bei einer besonders bevorzugten Weiterbildung des erfindungsgemäßen Verfahrens vorgeschlagen, bei der Bestimmung des Gesamtwärmetransfers auch sich über den Zeitablauf verändernde Umgebungstemperaturen mit einzubeziehen, so dass beispielsweise die an einem Leichenfundort auftretenden Temperaturänderungen, die sich durch den Sonnenstand und die Witterungsbedingungen ergeben, gleichfalls bei der Bestimmung des Gesamtwärmetransfers erfasst sind.

Der Modellkörper kann als Zylinder, als Quader oder ähnliches Volumenmodell aus entsprechenden Volumenelementen zusammengesetzt sein. Besonders bevorzugt entspricht der Modellkörper in seiner Gestalt und in seinen Proportionen der Gestalt und den Proportionen des zu begutachtenden Organismus. Dabei kann auch die Haltung und/oder die Lage des Organismus bei der Generierung des Modellkörpers mitberücksichtigt werden, so dass beispielsweise ein zusammengekrümmt liegender oder in hockender Stellung aufgefundener Leichnam gleichfalls als Modellkörper vorgegeben werden kann. Zu diesem Zweck wird beispielsweise vorgeschlagen, eine entsprechende Datenbank mit einer Vielzahl unterschiedlicher Modellkörper in unterschiedlicher Haltung und Lage vorzugeben. Entsprechend dem aufgefundenen, zu begutachtenden Organismus wird aus diesen verschiedenen Modellkörpern ein in seiner Haltung und in seiner Lage dem Organismus zumindest ähnlicher Modellkörper ausgewählt, der dann in seinen Proportionen durch entsprechende Volumenänderungen an die Gestalt des aufgefundenen Organismus angepasst wird.

Wird das erfindungsgemäße Verfahren zur Bestimmung des Todeszeitpunktes eines Menschen verwendet, wird die an dem verstorbenen Menschen gemessene Rektaltemperatur oder eine Temperatur an einem anderen oder mehreren Messorten am Mensch als Messwert bestimmt, der mit der theoretischen Temperaturänderung des Modellkörpers verglichen wird. Um eine vergleichbare Basis zu schaffen, wird die theoretische Temperaturänderung des Modellkörpers an einem Volumenelement des Modellkörpers bestimmt, der in seiner Lage im Modellkörper zumindest annähernd dem tatsächlichen Messort der Rektaltemperatur oder der anderen Temperaturen an der Leiche entspricht.

Nachfolgend wird die Erfindung anhand eines Ausführungsbeispieles unter Bezugnahme auf die Zeichnung näher erläutert. Darin zeigen:

Fig. 1     eine Schnittansicht eines aus einer Vielzahl von Volumenelementen gebildeten Modellkörpers eines Menschen, der in einzelne Körperkompartimente untergliedert ist, in dreidimensionaler Darstellung;

Fig. 2     einen Frontalschnitt durch den Modellkörper nach Fig. 1;

Fig. 3     eine perspektivische Darstellung der die Haut des Modellkörpers bildenden Volumenelemente;

Fig. 4     eine perspektivische Darstellung der das Fettgewebe des Modellkörpers bildenden Volumenelemente;

Fig. 5     eine perspektivische Darstellung der die Muskulatur des Modellkörpers bildenden Volumenelemente;

Fig. 6     eine perspektivische Darstellung der das Skelettsystem des Modellkörpers bildenden Volumenelemente;

Fig. 7        eine perspektivische Darstellung der das Gehirn, die Gesichtsweichteile und die Halseingeweide des Modellkörpers bildenden Volumenelemente;

Fig. 8        eine perspektivische Darstellung, der die Halseingeweide, die Lunge und den Mittelfellraum des Modellkörpers bildenden Volumenelemente;

Fig. 9        eine perspektivische Darstellung der die Oberbauchorgane, den Magen-Darm-Trakt, die Beckenorgane und das Nierenlager bildenden Volumenelemente;

Fig. 10       eine perspektivische Darstellung des Anfangstemperaturfeldes des Modellkörpers, wobei nur die linke Körperhälfte des Menschen als Modellkörper generiert worden ist;

Fig. 11       den Verlauf der inneren Wärmeproduktion bezogen auf die Zeit ausgehend von einem theoretischen Todeszeitpunkt;

Fig. 12       eine perspektivische Darstellung des Temperaturfeldes des Modellkörpers nach zwei Stunden Abkühlzeit;

Fig. 13       eine perspektivische Darstellung des Temperaturfeldes des Modellkörpers nach sieben Stunden Abkühlzeit;

Fig. 14       eine perspektivische Darstellung des Temperaturfeldes des Modellkörpers nach zweiunddreißig Stunden Abkühlzeit;

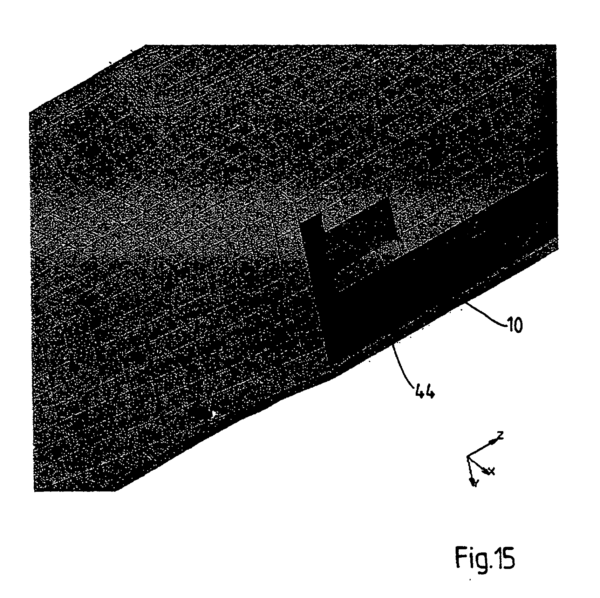

Fig. 15       eine vergrößerte Darstellung des Volumenelementes, an dem der Rektaltemperaturmesspunkt am Modellkörper vorgesehen ist;

Fig. 16       ein Temperatur-Zeit-Diagramm mit einer rektalen Abkühlkurve des Modellkörpers bei einer Umgebungstemperatur von 18°C;

Fig. 17       ein Temperatur-Zeit-Diagramm mit einer rektalen Abkühlkurve des Modellkörpers bei einer Umgebungstemperatur von 8°C;

Fig. 18       ein Temperatur-Zeit-Diagramm mit einer rektalen Abkühlkurve des Modellkörpers bei einer Umgebungstemperatur von 13°C, wobei der Modellkörper ein Körpergewicht von 61 kg und eine Körperlänge von 160 cm aufweist;

Fig. 19       ein Temperatur-Zeit-Diagramm mit einer rektalen Abkühlkurve des Modellkörpers bei einer Umgebungstemperatur von 13°C, wobei der Modellkörper ein Körpergewicht von 103 kg und eine Körpergröße von 190 cm aufweist;

Fig. 20       ein Temperatur-Zeit-Diagramm mit einer rektalen Abkühlkurve des Modellkörpers bei einer konstanten Umgebungstemperatur von 13°C und mit einer Abkühlkurve des Modellkörpers bei einem Temperatursprung der Umgebungstemperatur von 8°C auf 18°C;

Fig. 21       ein Temperatur-Zeit-Diagramm, in dem die Umgebungstemperaturverläufe für die Abkühlkurven von Fig. 20 gezeigt sind;

Fig. 22       ein Anfangstemperaturfeld des Modellkörpers mit konstanter externer Wärmeeinstrahlung;

Fig. 23       ein Temperaturfeld des Modellkörpers in Fig. 22 nach zwei Stunden Abkühldauer;

Fig. 24       ein Temperaturfeld des Modellkörpers in Fig. 22 nach sieben Stunden Abkühldauer;

Fig. 25       ein Temperaturfeld des Modellkörpers in Fig. 22 nach zweiunddreißig Stunden Abkühldauer; und

Fig. 26       ein Temperatur-Zeit-Diagramm, in dem eine rektale Abkühlkurve des Modellkörpers nach den Fig. 22 bis 25 bei einer Umgebungstemperatur von 13 °C dargestellt ist.

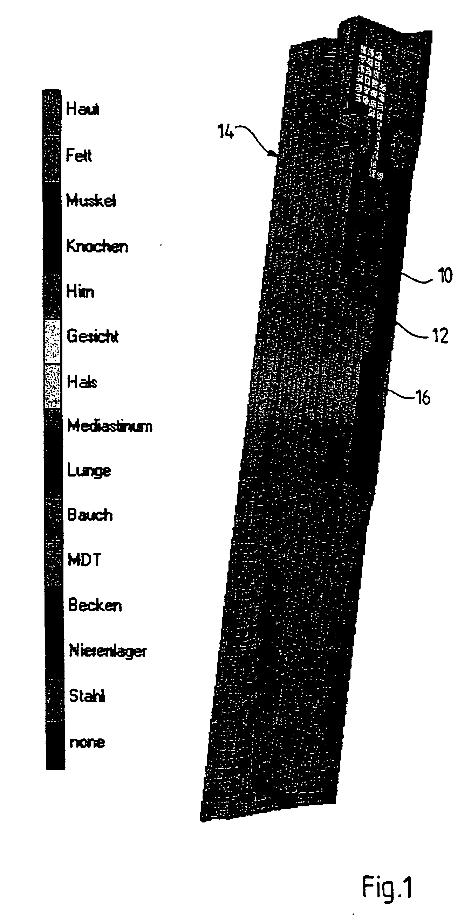

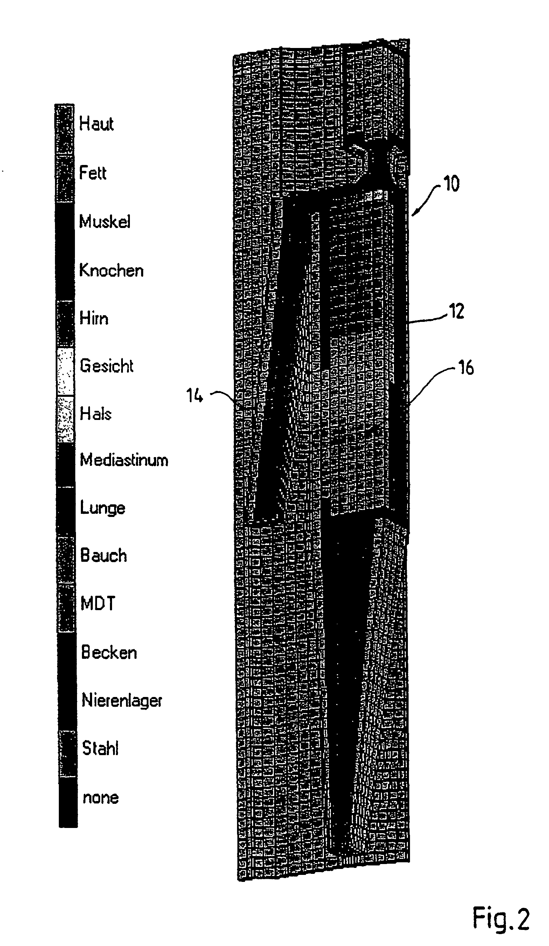

In den Fig. 1 und 2 ist ein halber Modellkörper 10 eines Menschen gezeigt, der aus einer Vielzahl von Volumenelementen 12 in einem Rechner modelliert worden ist. Der Modellkörper 10 liegt auf einer gleichfalls aus Volumenelementen 12 generierten Wanne 14 auf. Die Volumenelemente 12 sind im dargestellten Ausführungsbeispiel als Würfel ausgebildet, die an ihren als Knoten ausgebildeten Eckpunkten miteinander verbunden sind, wobei ein Wärmetransfer zwischen den einzelnen Volumenelementen 12 bestimmt werden kann, wie später noch erläutert wird.

Wie insbesondere den Fig. 1 und 2 zu entnehmen ist, ist der aus insgesamt 8328 Volumenelementen gebildete halbe Modellkörper 10 in einzelne, sogenannten Körperkompartimente 16 untergliedert, die in unterschiedlichen Helligkeitsstufen dargestellt sind. Die Körperkompartimente 16 sind aus Gruppen mehrerer Volumenelemente 12 gebildet, wobei sich die jeweils einer gemeinsamen Gruppe zugeordneten Volumenelemente 12 hinsichtlich ihrer thermodynamischen Eigenschaften gegenüber den Volumenelementen 12 anderer Gruppen unterscheiden. Auf diese Weise ist es möglich, den Modellkörper 10 in unterschiedliche Gewebetypen zu untergliedern, die sich durch unterschiedliche thermodynamische Eigenschaften auszeichnen. In Tabelle 1 ist die Untergliederung des Modellkörpers 10 in die einzelnen Körperkompartimente 16 dargestellt, wobei zwischen den einzelnen Gewebetypen unterschieden wird.

Tabelle 1:

| Untergliederung des Modellkörpers 10 in einzelne Körperkompartimente 16 | | |
|---|---|---|
| *Gewebe* | *Anzahl der Elemente* | *Volumenanteil am Modell* |
| *Haut* | 2740 | 10,3 % |
| *Fettgewebe* | 1798 | 26,2 % |
| *Muskulatur* | 2019 | 28,0 % |
| *Skelettsystem* | 680 | 10,0 % |
| *Gehirn* | 174 | 4,2% |
| *Gesichtsweichteile* | 91 | 2,3% |
| *Halseingeweide* | 14 | 0,2 % |
| *Mediastinum* | 104 | 2,6 % |
| *Lunge* | 220 | 5,0 % |
| *Oberbauchorgane* | 97 | 2,2 % |
| *Magen-Darm-Trakt* | 259 | 6,0 % |
| *Nierenlager* | 42 | 0,8 % |
| *Retroperitoneum* | 47 | 1,2 % |
| *Beckenorgane* | 43 | 1,0 % |
| Σ | 8328 | 100,0 % |

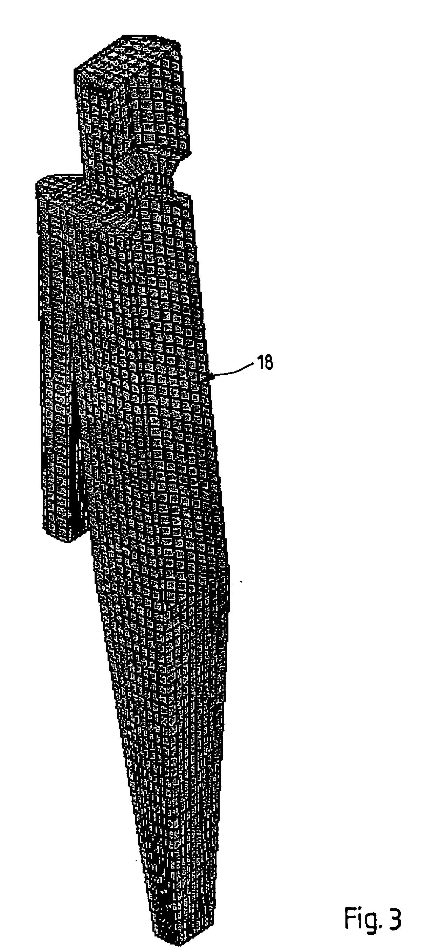

In den Fig. 3 bis 9 sind die unterschiedlichen Körperkompartimente des Modellkörpers 10 jeweils einzeln bzw. nach anatomischen Gesichtspunkten zusammengefasst dargestellt.

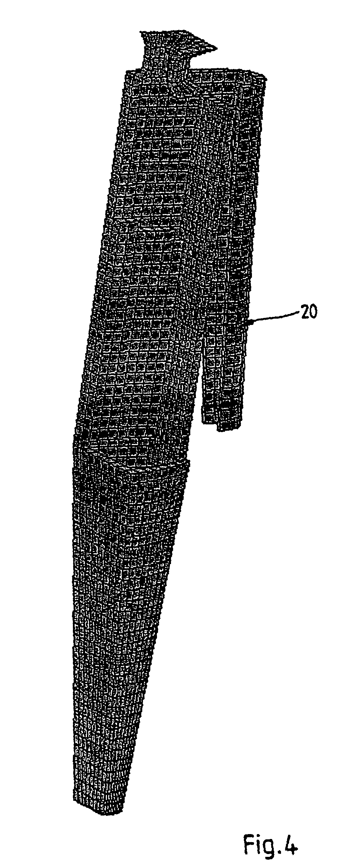

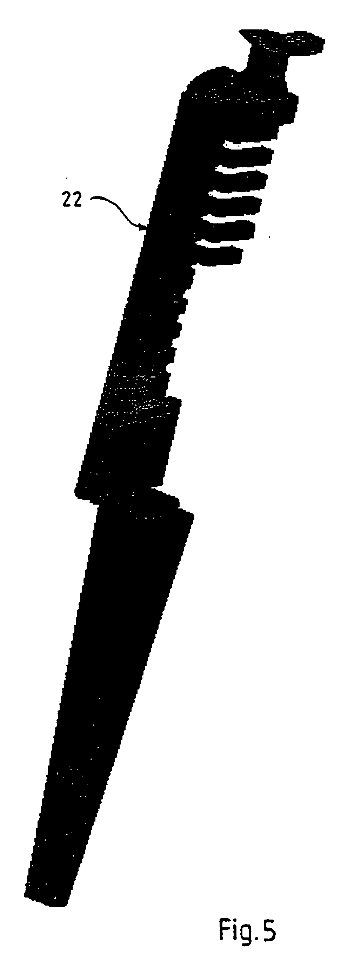

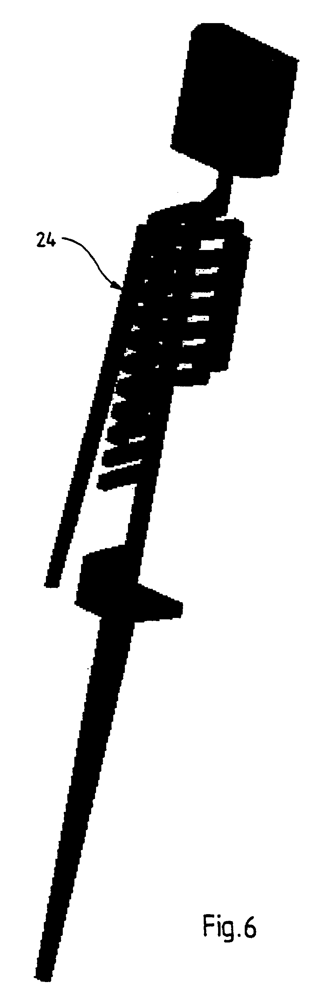

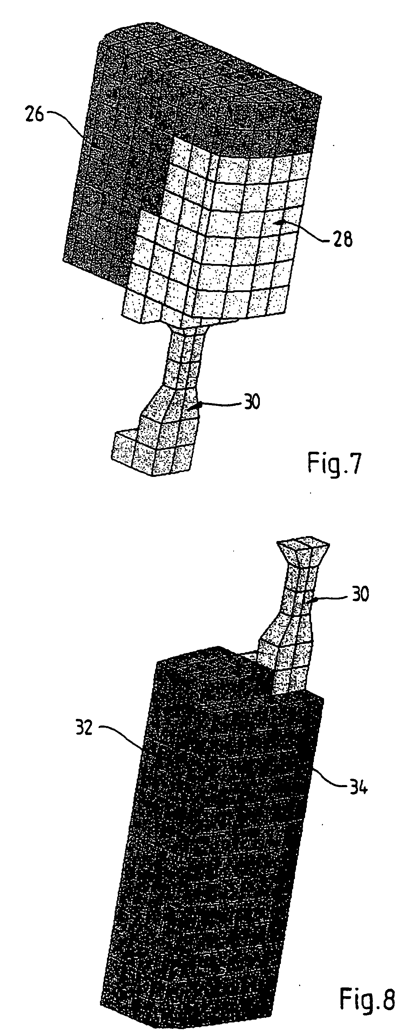

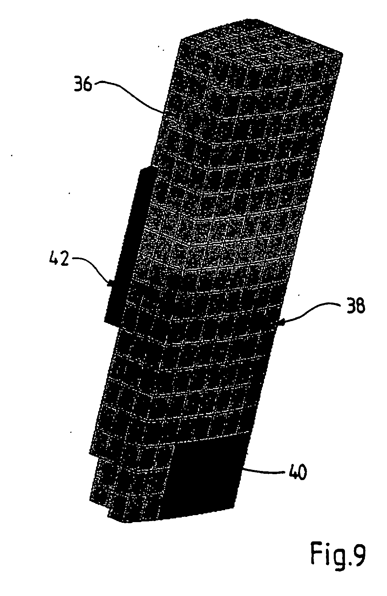

So zeigt Fig. 3 die Haut 18 des Modellkörpers 10, die beispielsweise aus 2740 Volumenelementen aufgebaut ist und einen Volumenanteil am Modellkörper 10 von etwa 10,3 % aufweist. Fig. 4 zeigt das Fettgewebe 20 des Modellkörpers 10, das aus 1798 Volumenelementen gebildet ist und einen Volumenanteil des Modellkörpers 10 von 26,2 % ausmacht. Fig. 5 zeigt die Muskulatur 22 des Modellkörpers 10, während Fig. 6 das aus den verschiedenen Knochen gebildete Skelettsystem 24 des Modellkörpers darstellt. In Fig. 7 sind mehrere Körperkompartimente zusammengefasst, so das Gehirn 26, die Gesichtsweichteile 28 und die Halseingeweide 30. Fig. 8 zeigt gleichfalls mehrere aus einer Vielzahl von Volumenelementen 12 gebildete Körperkompartimente, nämlich nochmals die Halseingeweide 30, die Lunge 32 sowie den Mittelfellraum 34. Fig. 9 zeigt schließlich die aus den Oberbauchorganen 36, dem Magen-Darm-Trakt 38, den Beckenorganen 40 und dem Nierenlager 42 gebildeten Körperkompartimente 16.

Der in den Fig. 1 und 2 gezeigte Modellkörper 10 ist aus diesen verschiedenen Körperkompartimenten 16 gebildet, wobei die verschiedenen Körperkompartimente 16 jeweils aus Volumenelementen zusammengesetzt sind, denen identische thermodynamische Eigenschaften zugeordnet werden. In der nachfolgenden Tabelle 2 sind die verschiedenen Körperkompartimente dem Gewebetyp nach aufgeführt, wobei die dem jeweiligen Gewebetyp eigenen thermodynamischen Eigenschaften, nämlich die thermische Konduktivität k, die spezifische Wärmekapazität c sowie die Massendichte $\rho$ aufgelistet sind. Dem Körperkompartiment Haut 18 ist darüber hinaus die Emissiviät $\varepsilon$ zugeordnet, um die Wärmeabstrahlung der Haut 18 darstellen zu können.

Tabelle 2:

| Auflistung der thermodynamischen Eigenschaften der Körperkompartimente | | | |
|---|---|---|---|
| Gewebe | $k\ [W\ m^{-1}\ ^{\circ}C^{-1}]$ | $c\ [J\ kg^{-1}\ ^{\circ}C^{-1}]$ | $\rho\ [kg/m^3]$ | $\varepsilon$ |
| Haut | 0,47 | 3680 | 1085 | 0,95 |
| Fettgewebe | 0,21 | 2300 | 920 | - |
| Muskulatur | 0,51 | 3800 | 1085 | - |
| Knochen | 0,75 | 1700 | 1357 | - |
| Hirn | 0,49 | 3850 | 1080 | - |
| Gesichtsweichteile | 0,51 | 3245 | 1056 | - |
| Halseingeweide | 0,48 | 3363 | 1006 | - |
| Mediastinum | 0,47 | 3375 | 1033 | - |
| Lunge | 0,28 | 3520 | 560 | - |
| Oberbauchorgane | 0,48 | 3730 | 1080 | - |
| Magen-Darm-Trakt | 0,46 | 3346 | 933 | - |
| Nierenlager | 0,39 | 3158 | 1026 | - |
| Retroperitoneum | 0,51 | 3800 | 1085 | - |
| Beckenorgane | 0,49 | 3350 | 1008 | - |

Basierend auf der Wärmeleitungsgleichung

$$c\rho\,\dot{T} - \lambda\Delta T = \dot{q}$$

wird nun die Temperaturänderung in dem aus den Volumenelementen 12 gebildeten Modellkörper 10 durch ein geeignetes nummerisches Verfahren bestimmt. Zu diesem Zweck wird die partielle Wärmeleitungsgleichung durch ein numerisches Verfahren gelöst, wobei die zuvor angegebenen thermodynamischen Eigenschaften, die thermische Konduktivität, die spezifische Wärmekapazität und die Massendichte, die innere Leistung zum Todeszeitpunkt und die Abfallrate der inneren Leistung sowie gegebenenfalls auch die Emissivität als Parameter der Wärmeleitungsgleichung verwendet werden. Darüber hinaus werden Randbedingungen für die Lösung der Wärmeleitungsgleichung vorgegeben, wie die Gestalt des Modellkörpers 10, die Temperaturverteilung im Modellkörper 10 zu Beginn der Auswertung und die Umgebungstemperatur.

In der nachfolgenden Tabelle 3 sind die verschiedenen Anfangstemperaturen für die verschiedenen Gliedmaßen, für den Kopf, den Hals und den Rumpf vorgegeben, die zu einem angenommenen theoretischen Todeszeitpunkt t = 0 als Anfangstemperaturniveau bzw. Anfangstemperaturfeld im Modellkörper 10 vorgegeben werden.

Tabelle 3:

| Anfangstemperaturfeld im Modellkörper 10 | | |
|---|---|---|
| Knoten ‚set' | Anzahl der Knoten | Temperatur $[^{\circ}C]$ |
| $Kopf_{aussen}$ | 355 | 35 |
| $Kopf_{mitte}$ | 254 | 36 |
| $Kopf_{innen}$ | 450 | 37,2 |
| $Hals_{aussen}$ | 60 | 35 |
| $Hals_{mitte}$ | 45 | 36 |
| $Hals_{innen}$ | 60 | 37,2 |
| $Rumpf_{aussen}$ | 816 | 35 |
| $Rumpf_{mitte}$ | 854 | 36 |
| $Rumpf_{innen}$ | 1873 | 37,2 |

Tabelle 3: (fortgesetzt)

| Anfangstemperaturfeld im Modellkörper 10 | | |
|---|---|---|
| *Knoten ‚set'* | *Anzahl der Knoten* | *Temperatur [°C]* |
| $Arm_{aussen}^1$ /$Arm_{innen}^1$ | 60 / 139 | 35 / 36 |
| $Arm_{aussen}^2$ / $Arm_{innen}^2$ | 36 / 132 | 34 / 35 |
| $Arm_{aussen}^3$ / $Arm_{innen}^3$ | 64 / 132 | 33 / 34 |
| $Arm_{aussen}^4$ / $Arm_{innen}^4$ | 64 / 132 | 32 / 33 |
| $Arm_{aussen}^5$ / $Arm_{innen}^5$ | 64 / 132 | 31 / 32 |
| $Arm_{aussen}^6$ / $Arm_{innen}^6$ | 64 / 132 | 30 / 31 |
| $Arm_{aussen}^7$ / $Arm_{innen}^7$ | 64 / 132 | 29 / 30 |
| $Arm_{aussen}^8$ / $Arm_{innen}^8$ | 64 / 132 | 28 / 29 |
| $Arm_{aussen}^9$ / $Arm_{innen}^9$ | 33 / 33 | 27 / 28 |
| $Bein_{aussen}^1$ / $Bein_{innen}^1$ | 107 / 345 | 35 / 36 |
| $Bein_{aussen}^2$ / $Bein_{innen}^2$ | 128 / 324 | 34 / 35 |
| $Bein_{aussen}^3$ / $Bein_{innen}^3$ | 128 / 324 | 33 / 34 |
| $Bein_{aussen}^4$ / $Bein_{innen}^4$ | 128 / 324 | 32 / 33 |
| $Bein_{aussen}^5$ / $Bein_{innen}^5$ | 128 / 324 | 31 / 32 |
| $Bein_{aussen}^6$ / $Bein_{innen}^6$ | 128 / 324 | 30 / 31 |
| $Bein_{aussen}^7$ / $Bein_{innen}^7$ | 128 / 324 | 29 / 30 |
| $Bein_{aussen}^8$ / $Bein_{innen}^8$ | 128 / 324 | 28 / 29 |
| $Bein_{aussen}^9$ / $Bein_{innen}^9$ | 81 / 81 | 27 / 28 |

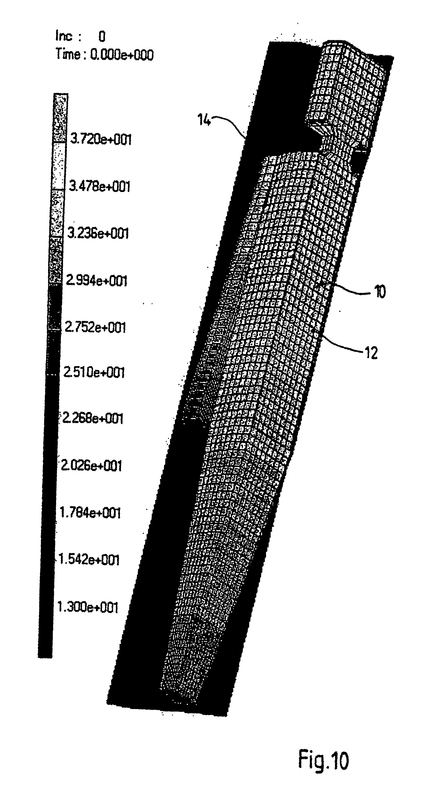

Diese Temperaturwerte können gegebenenfalls erhöht oder verringert werden, wenn bekannt ist, dass der zu begutachtende Mensch beispielsweise vor seinem Tod an Unterkühlung litt oder an einer schweren Infektion mit Fieber erkrankt war. In Fig. 10 ist der Modellkörper 10 mit diesem Anfangstemperaturfeld zum Zeitpunkt t = 0 dargestellt. Bei der Bestimmung der Temperaturänderung durch das numerische Verfahren wird nun die theoretische Temperaturänderung des Modellkörpers über das Volumen des Modellkörpers und/oder über die Zeit berechnet. Bei der Bestimmung dieser theoretischen Temperaturänderung des Modellkörpers über die Zeit werden zusätzlich weitere Randbedingungen mitberücksichtigt, nämlich der Konvektionskoeffizient und die Emmissivität, so dass die durch die Wärme des Modellkörpers verursachte freie Konvektion und Strahlungsabgabe bzw. -aufnahme mitberücksichtigt werden kann.

Dieser Wärmeverlust Q, der durch die Konvektion auftritt, wird durch die folgende Gleichung

$$Q = h_c \cdot (T_S - T_E)$$

berechnet, wobei $h_c$ der Konvektionskoeffizient, $T_S$ die Oberflächentemperatur und $T_E$ die Umgebungstemperatur sind. Der Konvektionskoeffizient $h_c$ liegt bei freier Konvektion bei 3,3 W/m². Der Konvektionskoeffizient $h_c$ kann gegebenenfalls, wenn bekannt war, dass an dem Leichenfundort beispielsweise Bodenwind herrschte, entsprechend erhöht werden. Des Weiteren werden bei der Bestimmung des durch Konvektion auftretenden Wärmeverlustes Q auch die Temperatur und die konvektive Wärmeabgabe der Wanne 14 mitberücksichtigt.

Neben der Konvektion wird auch die Wärmestrahlung des Modellkörpers und der unmittelbaren Umgebung (Wanne 14) mitberücksichtigt, indem die Emissivität ε der Haut 18 bzw. der Wanne 14 in die Bestimmung der theoretischen Temperaturänderung des Modellkörpers 10 einbezogen wird.

Eine weitere Randbedingung, die bei der Bestimmung der theoretischen Temperaturänderung einbezogen wird, ist die postmortale Wärmeproduktion des Organismus unmittelbar nach dem Tod des Organismus. Hierzu wird eine postmortale innere Wärmeleistung P bezogen auf die Zeit nach der folgenden Gleichung berechnet.

$$P(t) = P_0 \cdot \exp(-\alpha \cdot t)$$

Dabei definiert P die postmortale innere Wärmeleistung, $P_0$ den Anfangswert der inneren Leistung und $\alpha$ die Abfallrate der inneren Leistung. Der Anfangswert der inneren Leistung entspricht bei dem verwendeten Modell dem Grundumsatz eines Menschen, wobei der Grundumsatz gegebenenfalls in Abhängigkeit vom Alter des Menschen, von dessen Körperkonstitution oder dessen Größe variiert werden kann. Darüber hinaus wird der Gesamtgrundumsatz prozentual auf verschiedene Bereiche des Körpers aufgeteilt, wie die Tabelle 4 zeigt.

Tabelle 4:

| Prozentuale Verteilung der postmortalen inneren Wärmeleistung im Modellkörper 10. | | | |
|---|---|---|---|
| *Innere Anfangsleistung $P_0$* | *Kompartiment* | *Elementsets* | *Anzahl der Elemente* |
| 60 % | Rumpfkern | Lunge Mediastinum (Ober)Bauch MDT Nierenlager Becken(organe) | 765 |
| 18 % | Muskulatur | Muskulatur Retroperitoneum | 2066 |
| 17 % | Gehirn | Hirn | 174 |
| 5 % | Bindegewebe Skelett | Gesicht(sweichteile) Hals (eingeweide) Knochen | 785 |

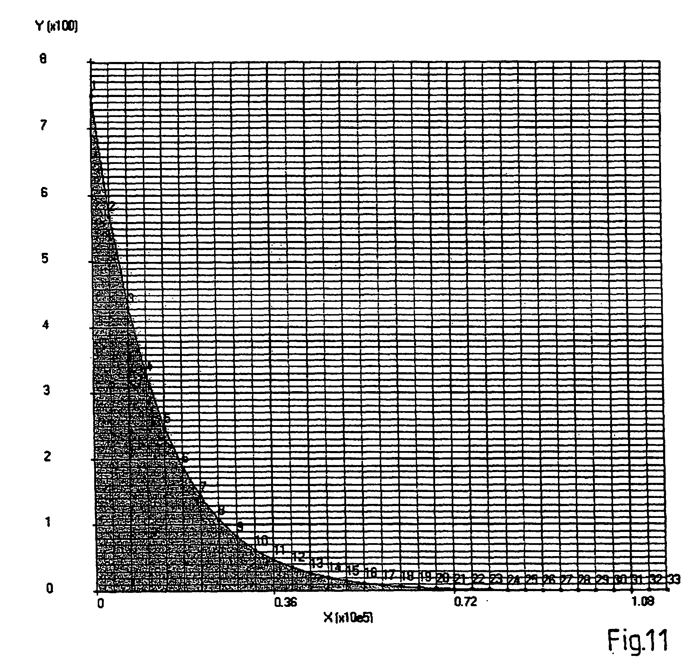

Als Abfallrate $\alpha$ wird ein Wert von 0,0000770164 s$^{-1}$ angesetzt, der einer Halbwertszeit von etwa 2,5 Stunden entspricht. Der sich aus der zuvor beschriebenen Gleichung ergebende Verlauf der postmortalen inneren Wärmeleistung des Rumpfkernes als Funktion der Zeit ist in Fig. 11 gezeigt. Hierbei ist zu erkennen, dass die innere Wärmeleistung exponentiell abfällt und nach etwa 24 Stunden bei 0 angelangt ist.

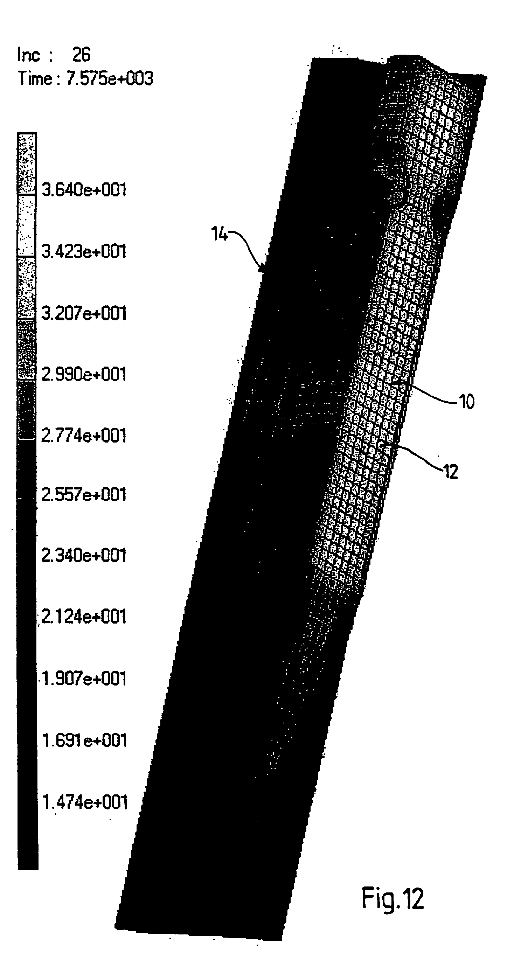

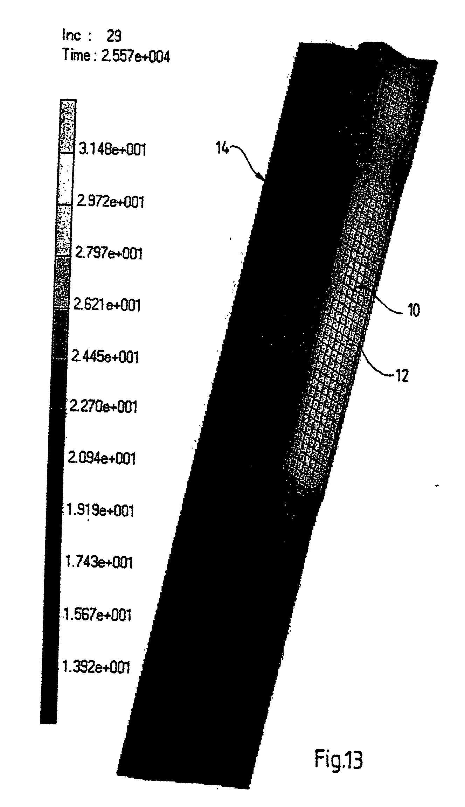

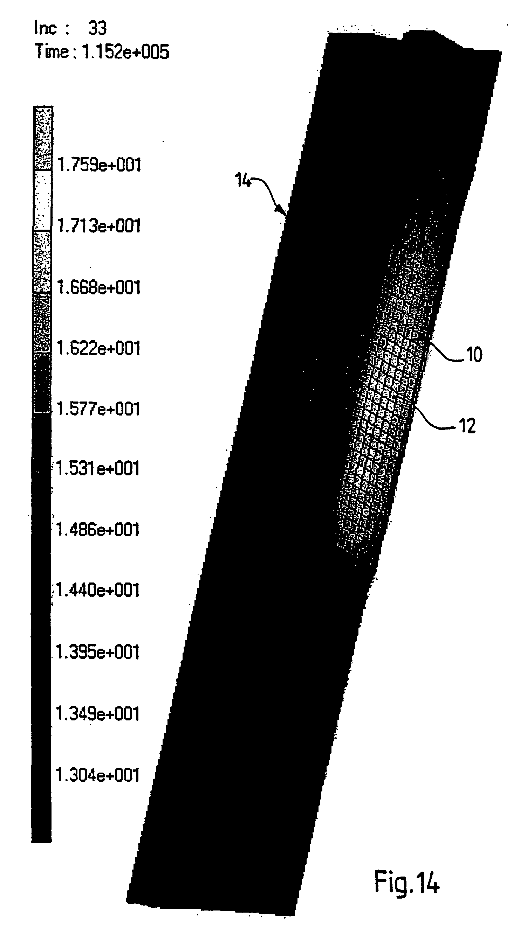

Ausgehend von diesen verschiedenen Anfangs- und Randbedingungen wird nun die Temperaturänderung des Modellkörpers 10 bestimmt. Hierbei werden kontinuierlich Momentaufnahmen des jeweiligen Temperaturzustandes des Modellkörpers 10 durch das numerische Verfahren ermittelt. In den Fig. 12 bis 14 sind Temperaturverteilungen des Modellkörpers 10 zu unterschiedlichen Zeitpunkten dargestellt, wobei helle Bereiche hohe Temperaturen und dunkle Bereiche niedrige Temperaturen angeben. So zeigt Fig. 12 die Temperaturverteilung des Modellkörpers 10 nach einer Abkühlzeit von etwa 2 Stunden, während Fig. 13 die Temperaturverteilung nach 7 Stunden Abkühlzeit und Fig. 14 die Temperaturverteilung nach 32 Stunden Abkühlzeit darstellt.

Basierend auf diesen Temperaturfeldern wird an einer definierten Stelle des Modellkörpers 10 die theoretische Temperaturänderung des Modellkörpers 10 erfasst und aufgenommen. Die Position dieser Messstelle 44 beim Modellkörper 10 entspricht in seiner Lage dem Rektalmesspunkt beim Menschen. Diese Messstelle 44 wird deshalb am Modellkörper 10 gewählt, um zu einem späteren Zeitpunkt aus der aufgenommenen theoretischen Temperaturänderung auf den Todeszeitpunkt zurückschließen zu können, da üblicherweise bei Leichenfunden oder Todesfällen aus Einfachheitsgründen die Rektaltemperatur gemessen wird. Prinzipiell wären aber auch andere und/oder mehrere Messorte vorstellbar und nutzbar.

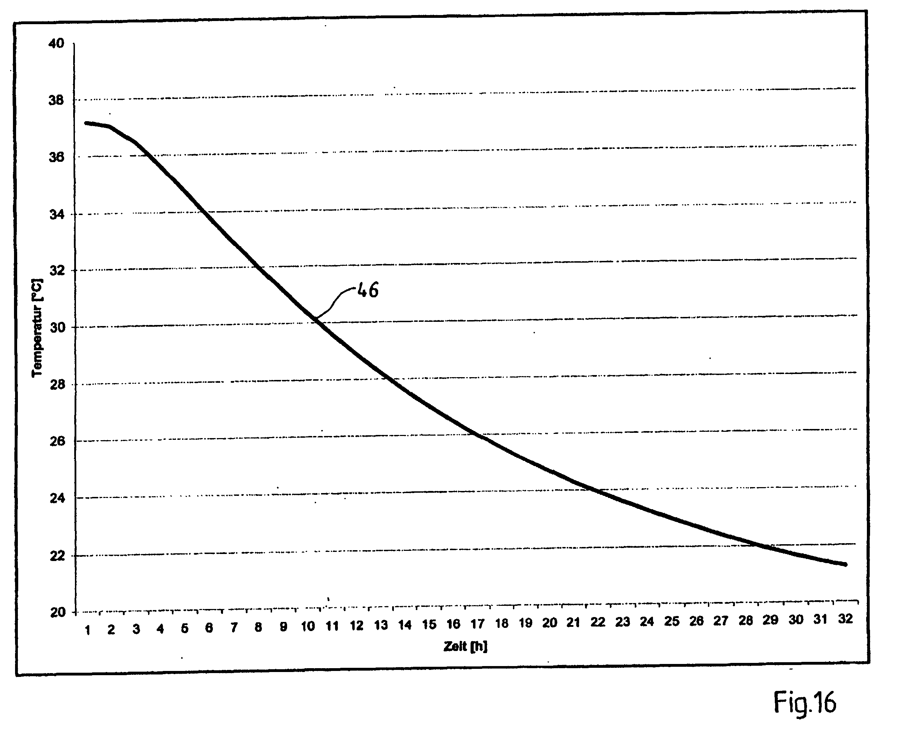

In den Fig. 16 bis 22 sind nun verschiedene Abkühlkurven in einem Temperatur-Zeit-Diagramm dargestellt, die an dieser Messstelle 44 aufgenommen worden sind.

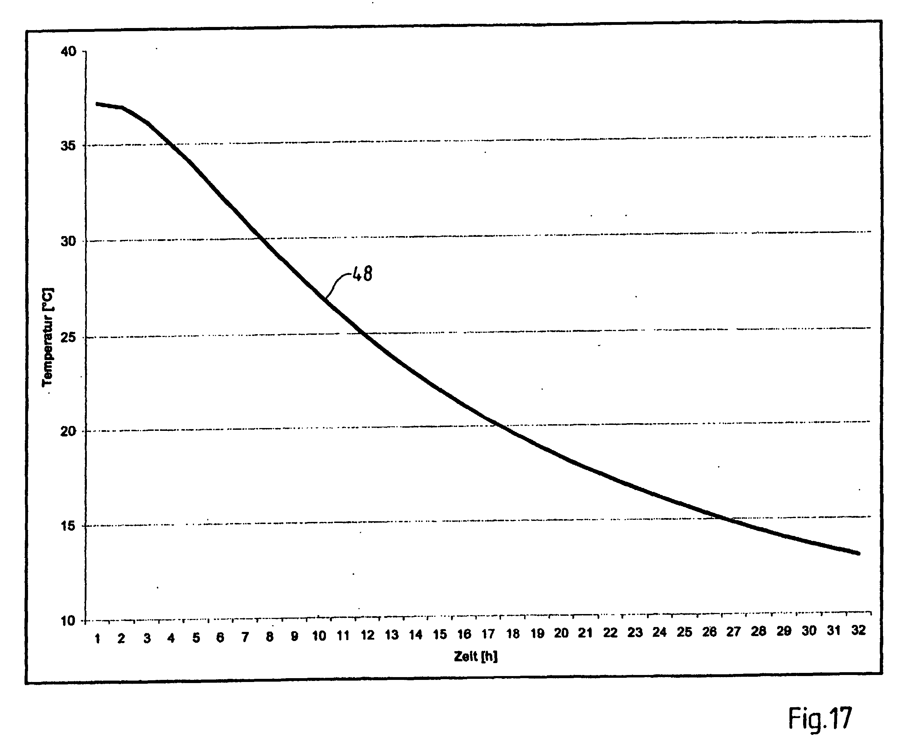

So zeigt Fig. 16 eine Abkühlkurve 46 des Modellkörpers 10 bei einer Umgebungstemperatur $T_E$ von 18°C. Die in Fig. 17 gezeigte Abkühlkurve 48 wurde bei einer Umgebungstemperatur $T_E$ von 8°C bestimmt.

Wie durch Vergleich der Abkühlkurven 46 und 48 in den Fig. 16 und 17 erkennbar ist, fällt die Abkühlkurve 48 bei einer Umgebungstemperatur von 8°C deutlich schneller ab als die Abkühlkurve 46 bei einer Umgebungstemperatur von 18°C.

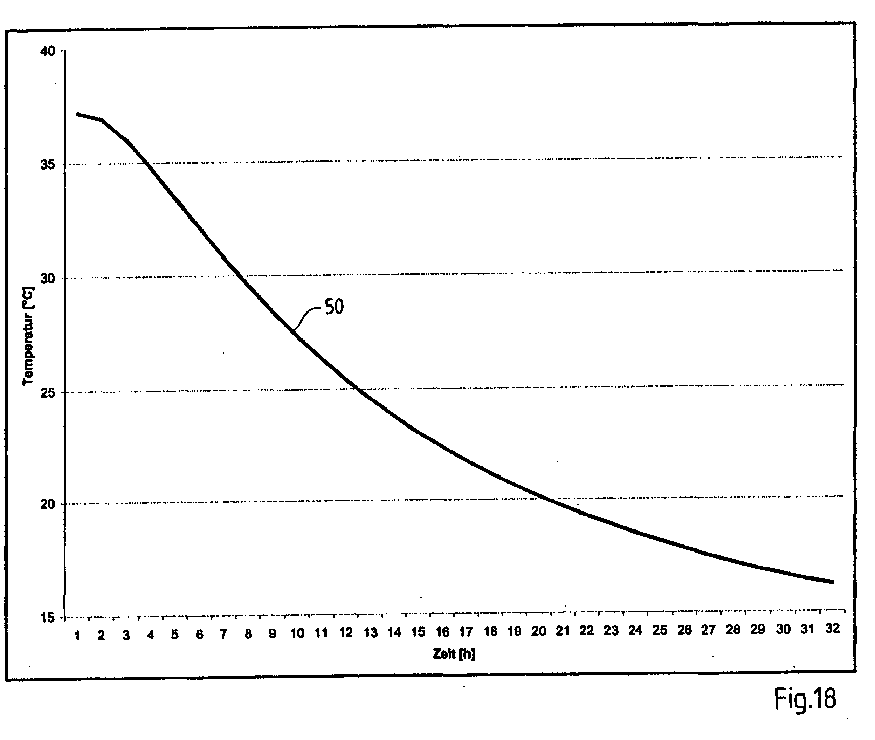

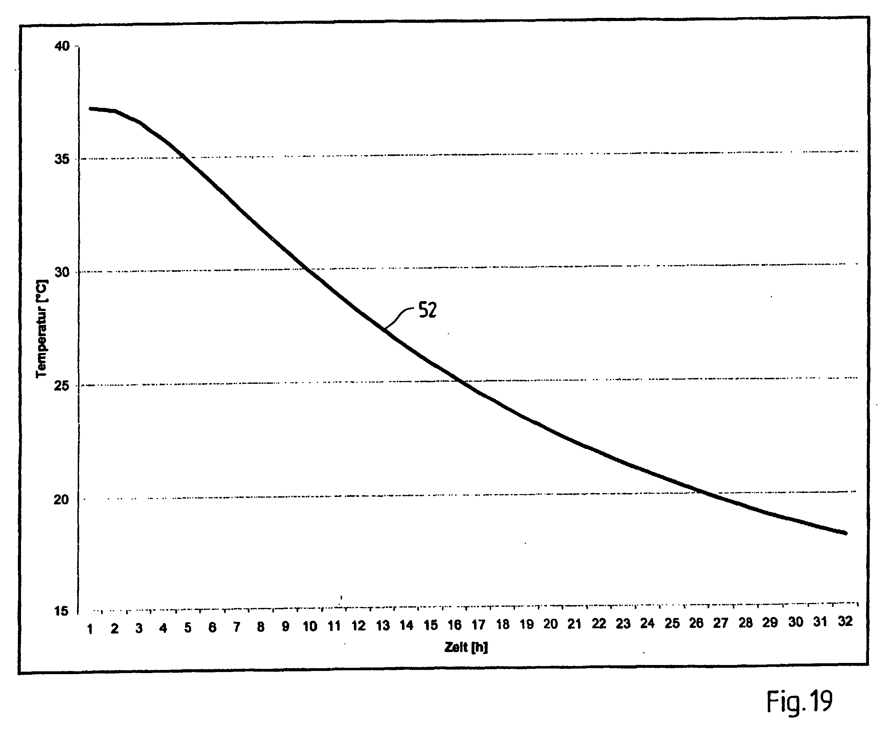

Bei den beiden in den Figuren 18 und 19 gezeigten Abkühlkurven 50 und 52 wurde eine Umgebungstemperatur $T_E$ von jeweils 13°C vorgegeben. Bei diesen Abkühlkurven 50 und 52 unterscheidet sich der Verlauf jedoch durch die vorgegebene Körpermasse des Modellkörpers 10 sowie die Körpergrösse. So betrifft die Abkühlkurve 50 in Fig. 18 einen Modellkörper 10 mit einem Körpergewicht von 61 kg und einer Körperlänge von 160 cm. Die in Fig. 19 gezeigte Abkühlkurve 52 betrifft dagegen einen Modellkörper 10 mit einem Körpergewicht von 103 kg und einer Körpergrösse von 190 cm.

Die beiden in den Figuren 18 und 19 gezeigten Abkühlkurven 50 und 52 belegen, dass der Modellkörper 10 mit größerer Masse weniger schnell abkühlt, als der Modellkörper 10 mit kleinerer Masse.

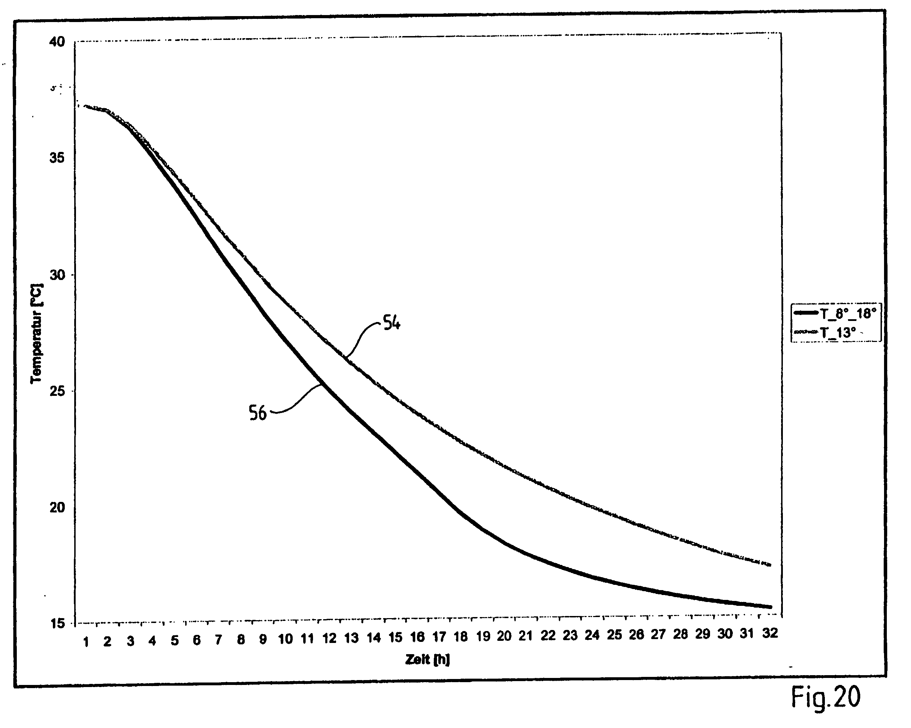

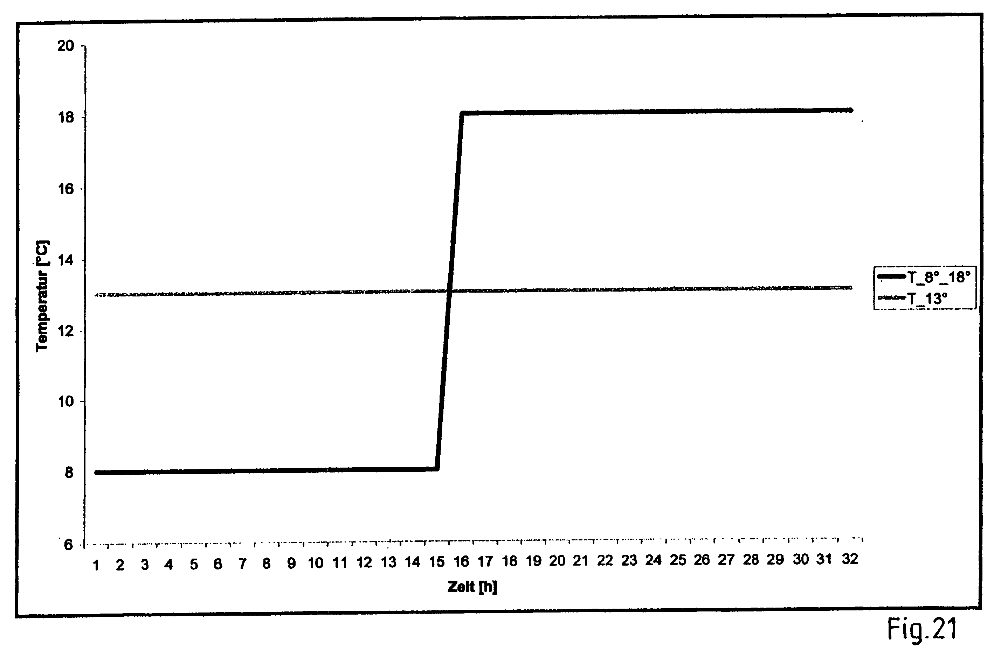

In den Figuren 20 und 21 ist der Einfluss sprunghafter Änderungen der Umgebungstemperatur auf die theoretische Temperaturänderung im Modellkörper 10 gezeigt. So zeigt Fig. 20 zwei Abkühlkurven 54 und 56 des Modellkörpers 10, wobei die Abkühlkurve 54 bei einer konstanten Umgebungstemperatur $T_E$ von 13°C ermittelt worden ist, während die Abkühlkurve 56 bei einer sprunghaften Temperaturänderung der Umgebungstemperatur $T_E$ von 8°C auf 18°C nach 16 Stunden zeigt. Die entsprechenden Temperaturverläufe sind in Fig. 21 dargestellt.

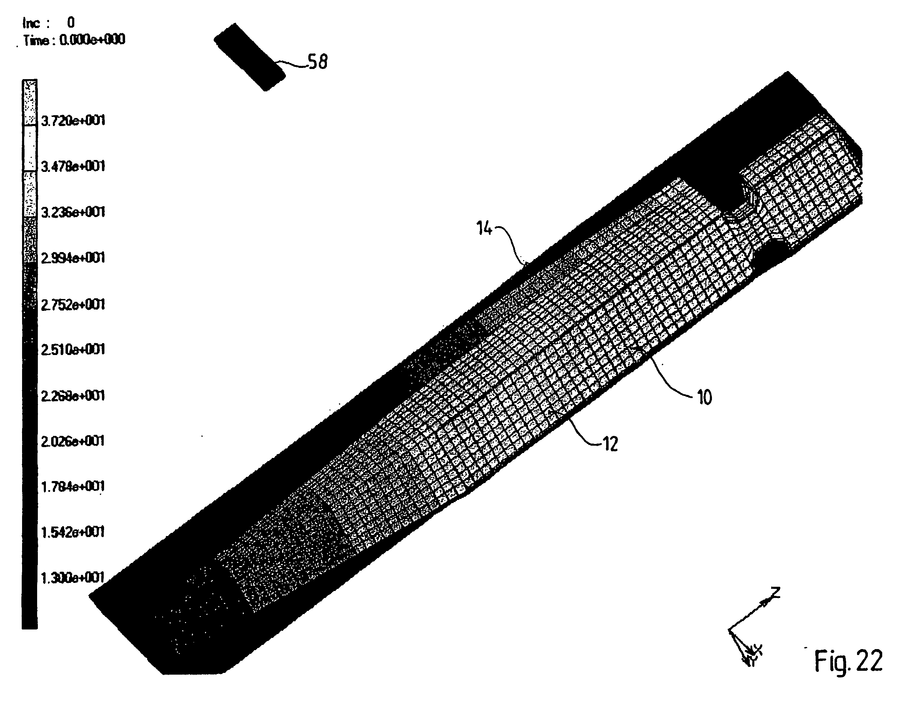

In den Figuren 22 bis 25 ist der Modellkörper 10 liegend auf der Wanne 14 dargestellt, wobei im Abstand von einem Meter zum Modellkörper 10 ein Wärmestrahler 58 angeordnet ist. Der Wärmestrahler 58 erzeugt eine konstante Strahlungstemperatur von 800°C, die einen Strahlungstransfer unmittelbar auf die Oberfläche des Modellkörpers 10 bewirkt. Durch diesen Wärmestrahler 58 soll beispielsweise ein benachbart zum Leichnam angeordneter Heizstrahler simuliert werden.

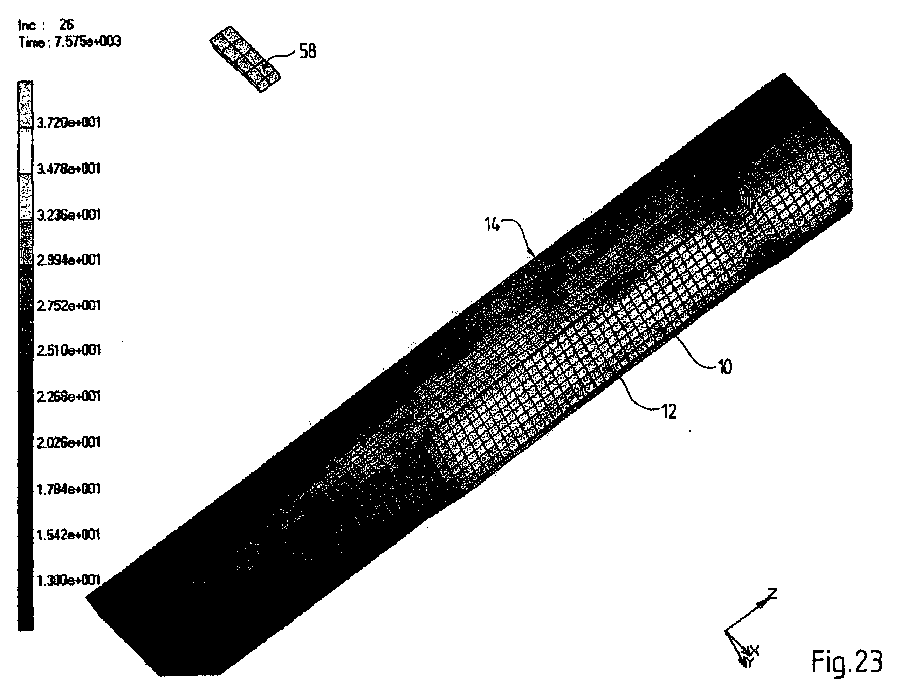

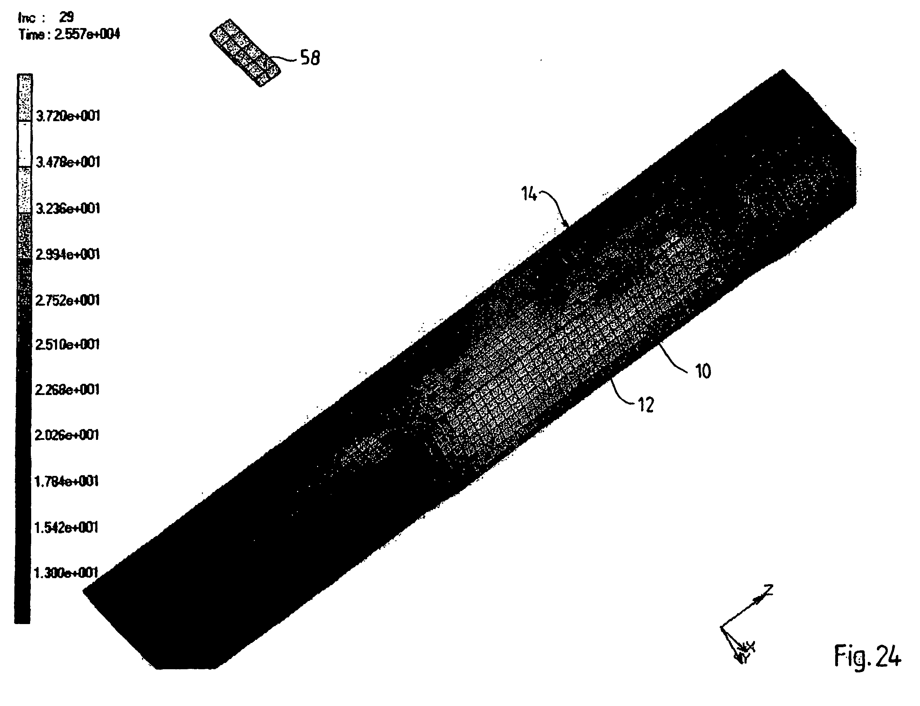

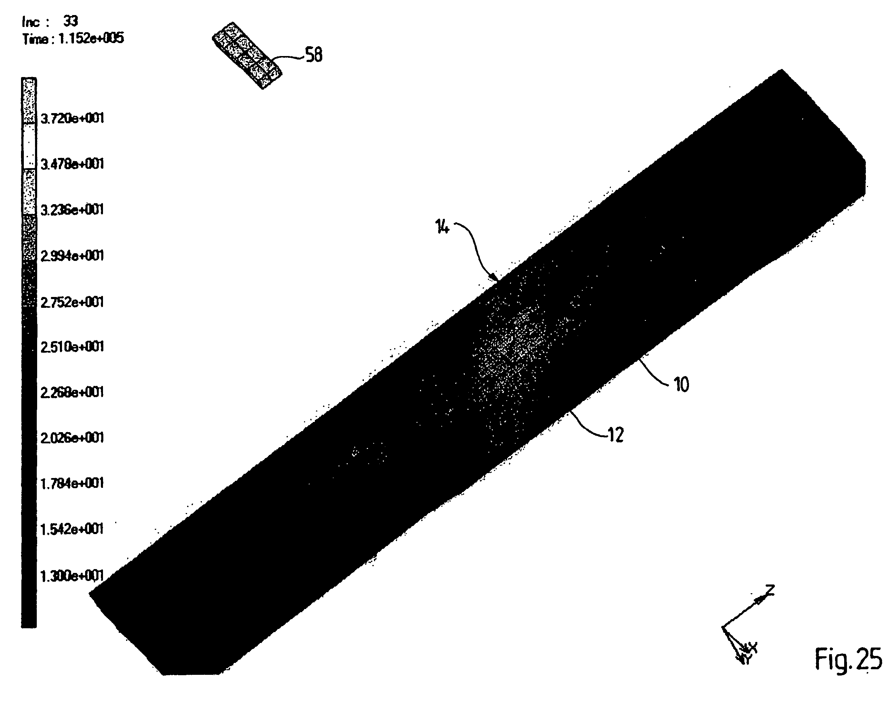

Wie den Temperaturniveaus in den Figuren 22 bis 25 zu entnehmen ist, wird die Oberfläche des Modellkörpers 10 durch den Wärmestrahler aufgewärmt, während der Modellkörper 10 von der Wanne 14 her langsam auskühlt. Auch hier wurden Temperaturfelder zu unterschiedlichen Zeitpunkten bestimmt, so zeigt Fig. 22 den Modellkörper 10 zum Zeitpunkt t = 0, Fig. 23 den Modellkörper 10 zum Zeitpunkt t = 2 Stunden, Fig. 24 den Modellkörper 10 zum Zeitpunkt t = 7 Stunden und schließlich Fig. 25 den Modellkörper 10 zum Zeitpunkt t = 32 Stunden.

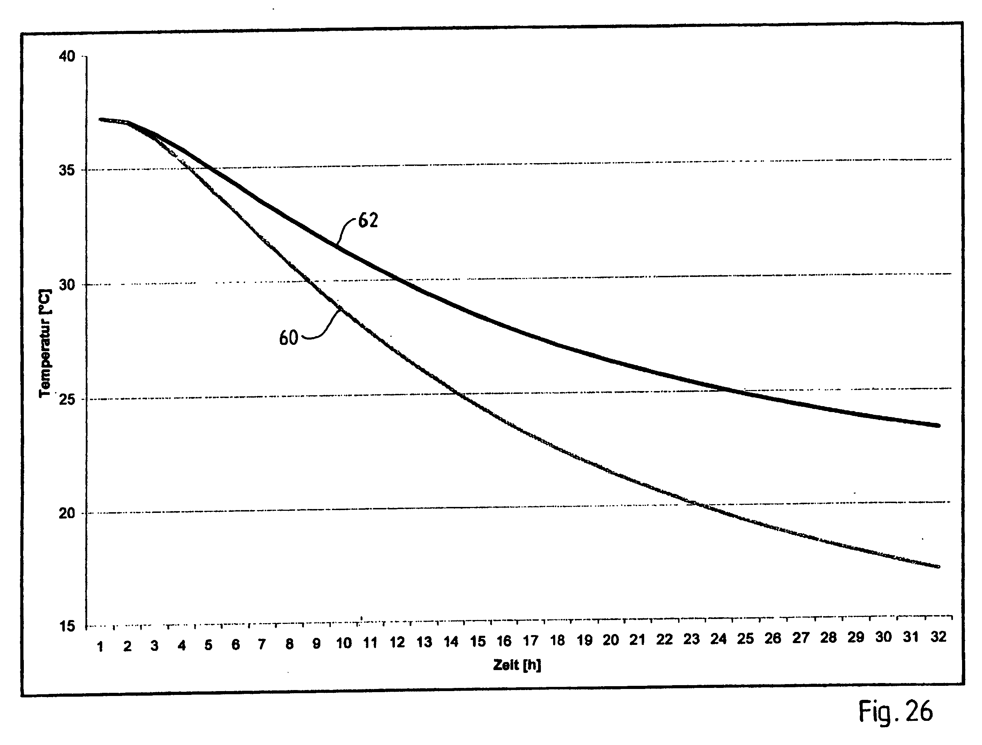

In Fig. 26 ist nun in einem Temperatur-Zeit-Diagramm der Verlauf einer Abkühlkurve 60 bei konstanter Umgebungstemperatur $T_E$ von 13°C aufgezeichnet. Die Abkühlkurve 62 zeigt dagegen die theoretische Temperaturänderung des Modellkörpers 10, die bei einer Umgebungstemperatur $T_E$ von 13°C auftritt, wenn gleichzeitig der Wärmestrahler 58 eine entsprechende Wärmestrahlung auf den Modellkörper 10 aussendet. Wie ein Vergleich der beiden Abkühlkurven 60 und 62 zeigt, verläuft die theoretische Temperaturänderung des Modellkörpers 10 bei Einwirkung der Wärmestrahlung im Vergleich zur Abkühlkurve 60 flacher, wodurch ersichtlich ist, dass der Modellkörper 10 durch die von der Wärmestrahlung verursachten Erwärmung weniger schnell auskühlt.

Auf der Grundlage dieser ermittelten Abkühlkurven ist es nun möglich, den tatsächlichen Todeszeitpunkt eines Menschen verglichen mit den bisher verwendeten Verfahren genauer und umfassender zu bestimmen.

So wird beispielsweise bei einem Leichenfund sowohl die Lage als auch die Position der Leiche festgehalten und mit Hilfe des Modellkörpers 10 und einer Modellumgebung als Basismodell generiert. Bei den dargestellten Ausführungsbeispielen wurde als Modellumgebung die Wanne 14 verwendet. Selbstverständlich kann anstelle der Wanne 14 eine entsprechend andersgestaltete Umgebung, beispielsweise ein Steinboden, Gras, Unterholz oder ähnliches realisiert werden, indem den Volumenelementen dieser Modellumgebung die entsprechenden thermodynamischen Eigenschaften zugeordnet werden. Darüber hinaus können Kleidungsstücke des Opfers, die unmittelbar am Körper anliegen und dessen Temperaturverhalten beeinflussen, aus den Volumenelementen 12 generiert werden. Anschließend kann der Modellkörper 10 in die entsprechende Lage positioniert werden, wobei der Modellkörper entsprechend den Körperproportionen, dem Gewicht und der Körpergrösse modelliert wird. Darüber hinaus können, wie zuvor bereits erläutert wurde, Konvektion, Konduktion, Wärmestrahlung und ähnliches mit in das Modellsystem eingegeben werden. Die Umgebungstemperatur kann zeitvariabel eingegeben werden. Außerdem können verschiedene Lagerungsphasen - wie z.B. bei einem Versuch der Leichenbeseitigung, beispielsweise durch Transport vom Tatort zum Ablageort, simuliert werden, indem das Temperaturfeld am Ende der einen Phase als Anfangstemperaturfeld der nächsten Phase berücksichtigt wird.

Nach Durchführung des numerischen Auswertungsverfahrens wird auf Grundlage dieser vorgegebenen Anfangs- und Randbedingungen an der die Rektalmeßstelle definierenden Meßstelle 44 eine entsprechende Abkühlkurve ermittelt, wie sie beispielsweise in einer der Figuren 16 bis 20 oder 22 bis 26 dargestellt ist. Mit dem Verfahren können auch Abkühlkurven an beliebigen anderen Lokalisationen aufgenommen und mit entsprechenden Messungen abgeglichen werden.

Sobald die Abkühlkurve bestimmt worden ist, wird in diese wenigstens eine tatsächlich gemessene Rektaltemperatur des Leichnams eingesetzt, wobei dann der dem gemessenen Temperaturwert in der theoretischen Temperaturänderung zugeordnete theoretische Meßzeitpunkt als tatsächlicher Meßzeitpunkt definiert wird. Da aus der Abkühlkurve und dem theoretischen Meßzeitpunkt auch der theoretische Todeszeitpunkt bekannt ist, der dem Nullpunkt zum Zeitpunkt t = 0 im Temperatur-Zeit-Diagramm entspricht, wird der theoretische Todeszeitpunkt als tatsächlicher Todeszeitpunkt definiert.

Mit dem erfindungsgemäßen Verfahren ist es also möglich, auf unterschiedlichste Anfangs- und Randbedingungen einzugehen, so dass eine sehr genaue und für verschiedenste Umgebungsbedingungen gültige Bestimmung der Abkühlgeschwindigkeit möglich ist.

**Patentansprüche**

1. Verfahren zur individuellen Bestimmung des Todeszeitpunktes eines biologischen Organismus, insbesondere eines Menschen, wobei bei dem Verfahren

   - eine von einem Anfangstemperaturniveau ausgehende, theoretische zeitliche Temperaturänderung (46-56, 60, 62) des Organismus vorgegeben wird, deren Anfangszeitpunkt einen theoretischen Todeszeitpunkt des Organismus definiert, und

- mindestens ein am Organismus nach dessen Ableben gemessener Temperaturwert mit der theoretischen Temperaturänderung (46-56, 60, 62) verglichen wird, wobei der dem gemessenen Temperaturwert in der theoretischen Temperaturänderung (46-46, 60, 62) zugeordnete theoretische Messzeitpunkt als tatsächlicher Messzeitpunkt der Temperaturmessung und der sich aus dem theoretischen Messzeitpunkt ergebende theoretische Todeszeitpunkt als tatsächlicher Todeszeitpunkt definiert wird,

**dadurch gekennzeichnet,**

- **dass** ein Modellkörper (10) des Organismus aus einer Vielzahl von Volumenelementen (12) generiert wird,
- **dass** zumindest einem Teil der Volumenelemente (12) jeweils wenigstens eine definierte thermodynamische Eigenschaft des Organismus zugeordnet wird,
- **dass** zumindest teilweise der Einzelwärmetransfer zwischen benachbart zueinander angeordneten Volumenelementen (12) ermittelt wird,
- **dass** basierend auf den thermodynamischen Eigenschaften der Volumenelemente (12) und basierend zumindest auf einem Teil der ermittelten Einzelwärmetransfers zwischen den Volumenelementen (12) als Gesamtwärmetransfer die Wärmeaufnahme und die Wärmeabgabe des Modellkörpers (10) bezogen auf die Zeit bestimmt wird und
- **dass** auf Grundlage des Gesamtwärmetransfers die theoretische zeitliche Temperaturänderung (46-56, 60, 62) des Organismus bestimmt wird.

2. Verfahren nach Anspruch 1,
**dadurch gekennzeichnet,**

- **dass** zumindest teilweise eine unmittelbare Modellumgebung (14) des Modellkörpers (10) aus Volumenelementen generiert wird,
- **dass** wenigstens einem Teil der Volumenelemente (12) der Modellumgebung (14) wenigstens eine definierte thermodynamische Eigenschaft der tatsächlichen Umgebung des Organismus zugeordnet wird,
- **dass** zumindest teilweise sowohl unter den Volumenelementen (12) der Modellumgebung (14) als auch zwischen den Volumenelementen (12) der Modellumgebung (14) und den Volumenelementen (12) des Modellkörpers (10) Einzelwärmetransfers bestimmt werden und
- **dass** der Gesamtwärmetransfer aus den thermodynamischen Eigenschaften der Volumenelemente (12) des Modellkörpers (10) und der Volumenelemente (12) der Modellumgebung (14) sowie aus den Einzelwärmetransfers zwischen den Volumenelementen (12) des Modellkörpers (10) und der Modellumgebung (14) bestimmt wird.

3. Verfahren nach Anspruch 1 oder 2,
**dadurch gekennzeichnet,**
**dass** den Volumenelementen (12) des Modellkörpers (10) als thermodynamische Eigenschaften die thermische Konduktivität, die spezifische Wärmekapazität und/oder die Massendichte zumindest eines Gewebetyps des Gewebes
des Organismus und/oder den Volumenelementen (12) der Modellumgebung (14) die thermische Konduktivität, die spezifische Wärmekapazität und/oder die Massendichte der die Umgebung bildenden Materialien zugeordnet werden.

4. Verfahren nach Anspruch 1, 2 oder 3,
**dadurch gekennzeichnet,**
**dass** den die Oberfläche des Modellkörpers (10) begrenzenden Volumenelementen (12) als eine weitere thermodynamische Eigenschaft die Emissivität zugeordnet wird.

5. Verfahren nach einem der Ansprüche 1 bis 4,
**dadurch gekennzeichnet,**

- **dass** der Modellkörper (10) in mehrere, Körperkompartimente (16) definierende Gruppen (18-42) von Volumenelementen (12) untergliedert wird und
- **dass** jeder Gruppe (18-42) von Volumenelementen (12) mindestens eine definierte thermodynamische Eigenschaft eines bestimmten Gewebetyps des Organismus zugeordnet wird.

**6.** Verfahren nach einem der Ansprüche 1 bis 5,
**dadurch gekennzeichnet,**
**dass** bei der Bestimmung des Gesamtwärmetransfers die am Modellkörper (12) auftretende Konvektion, Konduktion und/oder Strahlung mitberücksichtigt wird.

**7.** Verfahren nach Anspruch 6,
**dadurch gekennzeichnet,**
**dass** zur Berücksichtigung der Konvektion der Wärmeverlust Q durch die nachfolgende Gleichung

$$Q = h_c \cdot (T_S - T_E)$$

berechnet wird, wobei $h_c$ der Konvektionskoeffizient, $T_S$ die Oberflächentemperatur und $T_E$ die Umgebungstemperatur sind, und
**dass** der Konvektionskoeffizient $h_c$ in einem Bereich von etwa 3,0 bis 3,6 $W/m^2$, vorzugsweise bei 3,3 $W/m^2$ liegt, wenn ausschließlich freie Konvektion auftritt.

**8.** Verfahren nach Anspruch 7,
**dadurch gekennzeichnet,**
**dass** der Konvektionskoeffizient $h_c$ entsprechend der an der Oberfläche des Organismus aufgetretenen Strömungsbedingungen beim Modellkörper (10) und/oder bei der Umgebung (14) variiert wird.

**9.** Verfahren nach einem der Ansprüche 1 bis 8,
**dadurch gekennzeichnet,**

- **dass** bei der Bestimmung des Gesamtwärmetransfers eine postmortale Wärmeproduktion des Organismus mitberücksichtigt wird, wobei die anfängliche innere postmortale Wärmeproduktion des Organismus vorzugsweise der Höhe des Grundumsatzes des Organismus unmittelbar vor dessen Tod entspricht und
- **dass** die postmortale Wärmeproduktion ausgehend vom theoretischen Todeszeitpunkt linear oder exponentiell abnimmt.

**10.** Verfahren nach Anspruch 9,
**dadurch gekennzeichnet,**
**dass** die innere postmortale Wärmeproduktion entsprechend der Funktion

$$P(t) = P_0 \cdot \exp(-\alpha \cdot t)$$

bestimmt wird, wobei P die postmortale innere Wärmeleistung, $P_0$ den Anfangswert der inneren Leistung und $\alpha$ die Abfallrate der inneren Leistung definiert und die Abfallrate $\alpha$ vorzugsweise einen Wert in einem Bereich von 0,00002 $s^{-1}$ bis 0,00020 $s^{-1}$, besonders bevorzugt einen Wert von 0,000077 $s^{-1}$ aufweist.

**11.** Verfahren nach Anspruch 9 oder 10,
**dadurch gekennzeichnet,**

- **dass** die innere Wärmeproduktion prozentual auf mehrere, Körperkompartimente (16) des Organismus bildende Gruppen (18-42) von Volumenelementen unterschiedlich verteilt wird und
- **dass**, wenn der Todeszeitpunkt eines Menschen mit dem Verfahren bestimmt wird, 55 bis 65 % des Grundumsatzes des Menschen auf den Rumpfkern, 16 bis 20 % des Grundumsatzes auf die Muskulatur, 15 bis 19 % des Grundumsatzes auf das Gehirn und 4 bis 6 % des Grundumsatzes auf die Knochen und das Bindegewebe des Menschen entfallen.

**12.** Verfahren nach einem der vorhergehenden Ansprüche,
**dadurch gekennzeichnet,**
**dass** bei der Bestimmung des Gesamtwärmetransfers auch sich über den Zeitablauf verändernde Umgebungstemperaturen mitberücksichtigt werden.

**13.** Verfahren nach einem der vorhergehenden Ansprüche,

**dadurch gekennzeichnet,**
**dass** eine externe Einstrahlung mitberücksichtigt wird.

**14.** Verfahren nach einem der vorhergehenden Ansprüche,
**dadurch gekennzeichnet,**
**dass** verschiedene aufeinanderfolgende Lagerungsphasen mit unterschiedlichen Umgebungsbedingungen erfasst werden, indem das ermittelte Temperaturfeld am Ende der einen Phase als Anfangstemperaturfeld für die nächstfolgende Phase verwendet wird.

**15.** Verfahren nach einem der vorhergehenden Ansprüch
**dadurch gekennzeichnet,**
**dass** der Modellkörper (10) in seiner Gestalt, in seinen Proportionen, in seiner Haltung und/oder in seiner Lage zumindest annähernd entsprechend der Gestalt, den Proportionen, der Haltung und/oder der Lage des zu begutachtenden Organismus generiert wird.

**16.** Verfahren nach einem der vorhergehenden Ansprüche,
**dadurch gekennzeichnet,**

- **dass** das Verfahren zur Bestimmung des Todeszeitpunktes eines Menschen verwendet wird,
- **dass** zum Vergleich mit der theoretischen Temperaturänderung (46-56, 60, 62) des Modellkörpers (10) die Rektaltemperatur oder Temperaturen an anderen Messorten des verstorbenen Menschen gemessen werden und
- **dass** der in Form eines Menschen generierte Modellkörper (10) ein Volumenelement aufweist, das entsprechend seiner Lage im Modellkörper (10) als Vergleichsmesspunkt (44) für die Rektaltemperatur oder Temperaturen an anderen Messorten verwendet wird, wobei die theoretische Temperaturänderung (46-56, 60, 62) an diesem Volumenelement zur Bestimmung des Todeszeitpunktes mit der gemessenen Rektaltemperatur verglichen wird.

**17.** Verfahren nach einem der vorhergehenden Ansprüche,
**dadurch gekennzeichnet,**

- **dass** die den Gesamtwärmetransfer des Modellkörpers (10) beschreibende partielle Wärmeleitungsdifferentialgleichung mit Hilfe eines numerischen Verfahrens gelöst wird, wobei die thermodynamischen Eigenschaften als Parameter in die Wärmeleitungsdifferentialgleichung eingesetzt werden, und
- **dass** bei der numerischen Lösung der partiellen Wärmeleitungsdifferentialgleichung das Anfangstemperaturniveau als Anfangsbedingung und eine Umgebungstemperatur als Randbedingungen vorgegeben werden.

**Claims**

**1.** A method of individually determining the time of death of a biological organism, particularly of a human being, in which

- a theoretical change in temperature over time (46-56, 60, 62), starting from an initial temperature level, of the organism is predetermined, the starting point of which defines a theoretical time of death of the organism, and
- at least one temperature value measured on the organism after its demise is compared with the theoretical change in temperature (46-56, 60, 62), whereby the theoretical time of measurement associated with the measured temperature value in the theoretical change in temperature (46-56, 60, 62) is defined as the actual time of measurement of the temperature measurement and the theoretical time of death resulting from the theoretical time of measurement is defined as the actual time of death,

**characterised in that**

- a model body (10) of the organism is generated from a plurality of volume elements (12),
- that at least one respective defined thermodynamic property of the organism is associated with at least one portion of the volume elements (12),
- that the individual heat transfer between volume elements (12) disposed adjacent to one another is at least partially determined,

- that based on the thermodynamic properties of the volume elements (12) and based on at least a proportion of the determined individual heat transfers between the volume elements (12), the heat absorption and the heat emission, constituting the total heat transfer, of the model body (10) is determined with respect to time and
- that the theoretical change in temperature over time (46-56, 60, 62) of the organism is determined on the basis of the total heat transfer.

2. A method as claimed in Claim 1, **characterised in that**

- a direct model environment (14) of the model body (10) is generated, at least in part, from volume elements,
- that at least one defined thermodynamic property of the actual environment of the organism is associated with at least a proportion of the volume elements (12) of the model environment (14),
- that heat transfers both between the volume elements (12) of the model environment (14) and also between the volume elements (12) of the model environment (14) and the volume elements (12) of the model body (10) are determined, at least in part, and
- that the total heat transfer is determined from the thermodynamic properties of the volume elements (12) of the model body (10) and of the volume elements (12) of the model environment (14) and from the individual heat transfers between the volume elements (12) of the model body (10) and of the model environment (14).

3. A method as claimed in Claim 1 or 2, **characterised in that** the thermal conductivity, the specific thermal capacity and/or the density of at least one tissue type of the tissue of the organism, constituting thermodynamic properties, are associated with the volume elements (12) of the model body (10) and/or the thermal conductivity, the specific thermal capacity and/or the density of the materials constituting the environment are associated with the volume elements (12) of the model environment (14).

4. A method as claimed in Claim 1, 2 or 3, **characterised in that** the emissivity, constituting a further thermodynamic property, is associated with the volume elements (12) defining the surface of the model body (10).

5. A method as claimed in one of Claims 1 to 4, **characterised in that**

- the model body (10) is subdivided into a plurality of groups (18-42) of volume elements (12) defining body compartments (16) and
- that associated with each group (18-42) of volume elements (12) there is at least one defined thermodynamic property of a predetermined tissue type of the organism.

6. A method as claimed in one of Claims 1 to 5, **characterised in that** when determining the total heat transfer, the convection, conduction and/or radiation occurring at the model body (12) is also taken into consideration.

7. A method as claimed in Claim 6, **characterised in that** in order to take the convection into consideration, the heat loss Q is calculated by the following equation

$$Q = h_c \cdot (T_S - T_E)$$

wherein $h_c$ is the coefficient of convection, $T_S$ is the surface temperature and $T_E$ is the environmental temperature and
that the coefficient of convection $h_c$ is in a range of about 3.0 to 3.6 $W/m^2$, preferably 3.3 $W/m^2$, when exclusively free convection occurs.

8. A method as claimed in Claim 7, **characterised in that** the coefficient of convection $h_c$ is varied at the model body (10) and/or at the environment (14) corresponding to the flow conditions occurring at the surface of the organism.

9. A method as claimed in one of Claims 1 to 8, **characterised in that**

- when determining the total heat transfer, a postmortal heat production of the organism is also taken into consideration, whereby the initial internal postmortal heat production of the organism preferably corresponds to the basal metabolic rate of the organism directly before its death and
- that the postmortal heat production decreases, starting from the theoretical time of death, linearly or exponentially.

**10.** A method as claimed in Claim 9, **characterised in that** the internal postmortal heat production is determined in accordance with the function

$$P(t) = P_O \cdot \exp(-\alpha \cdot t)$$

wherein P defines the postmortal internal heat production, $P_O$ defines the initial value of the internal production and $\alpha$ defines the decay rate of the internal production and the decay rate $\alpha$ preferably has a value in the range of 0.00002 s$^{-1}$ to 0.00020 s$^{-1}$, particular preferably a value of 0.000077 s$^{-1}$.

**11.** A method as claimed in Claim 9 or 10, **characterised in that**

- the internal heat production is distributed in different percentages over a plurality of groups (18-42) of volume elements constituting body compartments (16) of the organism and
- that when the time of death of a human body is determined with the method, 55 to 65% of the basal metabolic rate of the human being is associated with the thorax core, 16 to 20% of the basal metabolic rate is associated with the muscular system, 15 to 19% of the basal metabolic rate is associated with the brain and 4 to 6% of the basal metabolic rate is associated with the bones and the connective tissue of the human body.

**12.** A method as claimed in one of the preceding claims, **characterised in that** when determining the total heat transfer, the environmental temperatures, which change over the course of time, are also taken into consideration.

**13.** A method as claimed in one of the preceding claims, **characterised in that** external radiation is also taken into consideration.

**14.** A method as claimed in one of the preceding claims, **characterised in that** different, successive storage phases with different environmental conditions are detected, the temperature field determined at the end of one phase being used as the starting temperature field for the subsequent phase.

**15.** A method as claimed in one of the preceding claims, **characterised in that** the model body (10) is generated as regards its shape, its proportions, its attitude and/or its position to correspond at least approximately to the shape, the proportions, the attitude and/or the position of the organism on which the opinion is to be given.

**16.** A method as claimed in one of the preceding claims, **characterised in that**

- the method of determining the time of death of a human body is used,
- that for the purpose of comparison with the theoretical change in temperature (46-56, 60, 62) of the model body (10), the rectal temperature or temperatures at other measuring locations of the deceased human being are measured and
- that the model body (10) generated in the form of a human being has a volume element, which is used, in accordance with its position in the model body (10) as a comparative measuring point (44) for the rectal temperature or temperatures at other measuring locations, whereby the theoretical change in temperature (46-56, 60, 62) at this volume element is compared with the measured rectal temperature in order to determine the time of death.

**17.** A method as claimed in one of the preceding claims, **characterised in that**

- the partial thermal conduction differential equation describing the total heat transfer of the model body (10) is solved with the aid of a numerical method, whereby the thermodynamic properties are inserted as parameters into the thermal conductivity differential equation, and
- that the initial temperature level is predetermined as a starting condition and an environmental temperature is predetermined as a boundary condition in the numerical solution of the partial thermal conductivity differential equation.

**Revendications**

1. Procédé permettant de déterminer individuellement l'heure ou instant du décès d'un organisme biologique, en particulier d'un être humain, dans lequel durant le procédé

   - il est prédéterminé une modification temporelle théorique de la température (46-56, 60, 62) de l'organisme partant d'un niveau de température initial, dont l'heure initiale définit une heure théorique du décès de l'organisme, et
   - au moins une valeur de température mesurée sur l'organisme après son décès est comparée avec la modification théorique de la température (46-56, 60, 62), l'heure théorique de mesure attribuée à la valeur de température mesurée lors du changement de température théorique (46-46, 60, 62) étant définie comme l'heure effective de la mesure pour la mesure de la température et l'heure théorique du décès résultant de l'heure théorique de la mesure et étant définie comme l'heure effective du décès,

   **caractérisé en ce**

   - **qu'**un corps type (10) de l'organisme est généré à partir d'une pluralité d'éléments volumiques (12),
   - **qu'**au moins une propriété thermodynamique définie de l'organisme est à chaque fois attribuée au moins à une partie des éléments volumiques (12),
   - **qu'**on détermine qu'au moins partiellement le transfert thermique individuel entre éléments volumiques (12) voisins les uns par rapport aux autres,
   - **qu'**on détermine l'acquisition et l'abandon de chaleur du corps type (10) rapporté au temps, en se reposant sur les propriétés thermodynamiques des éléments volumiques (12) et en se reposant au moins sur une partie des transferts thermiques individuels entre les éléments volumiques (12), comme étant le transfert thermique global,
   - **que** la modification de température temporelle théorique (46-56, 60, 62) de l'organisme est déterminée en se basant sur le transfert thermique global.

2. Procédé selon la revendication 1, **caractérisé en ce**

   - **qu'**un environnement type (14) immédiat du corps type (10) est généré au moins partiellement à partir des éléments volumiques,
   - **qu'**au moins une propriété thermodynamique définie de l'environnement effectif de l'organisme est attribuée à au moins une partie aux éléments volumiques (12) de l'environnement type (14),
   - **que** des transferts thermiques individuels sont déterminés au moins partiellement entre les éléments volumiques (12) de l'environnement type (14) mais également entre les éléments volumiques (12) de l'environnement type (14) et les éléments volumiques (12) du corps type (10) et
   - **que** le transfert thermique global est déterminé à partir des propriétés thermodynamiques des éléments volumiques (12) du corps type (10) et des éléments volumiques (12) de l'environnement type (14) ainsi qu'à partir des transferts thermiques individuels entre les éléments volumiques (12) du corps type (10) et ceux de l'environnement type (14).

3. Procédé selon la revendication 1 ou 2 **caractérisé en ce que** la conductivité thermique, la capacité thermique spécifique et/ou la densité de masse au moins d'un type de tissu de l'organisme sont attribuées aux éléments volumiques (12) du corps type (10) comme propriétés thermodynamiques et/ou la conductivité thermique, la capacité thermique spécifique et/ou la densité de masse des matériaux formant l'environnement sont attribuées aux éléments volumiques (12) de l'environnement type (14).

4. Procédé selon la revendication 1, 2 ou 3, **caractérisé en ce que** l'émissivité est attribuée aux éléments volumiques (12) limitant la surface du corps type (10) comme étant une autre propriété thermodynamique.

5. Procédé selon l'une quelconque des revendications 1 à 4, **caractérisé en ce**

   - **que** le corps type (10) est sous-divisé en plusieurs groupes (18-42) d'éléments volumiques (12) définissant des compartiments corporels (16) et
   - **qu'**au moins une propriété thermodynamique définie d'un type de tissu déterminé de l'organisme est attribuée à chaque groupe (18-42) d'éléments volumiques (12).

**6.** Procédé selon l'une quelconque des revendications 1 à 5,
   **caractérisé en ce que**
   l'on prend en compte la convection, la conduction et/ou le rayonnement apparaissant sur le corps type (12) lors de la détermination du transfert thermique global.

**7.** Procédé selon la revendication 6,
   **caractérisé en ce que**

   - pour la prise en compte de la convection, la perte de chaleur Q est calculée par l'intermédiaire de l'équation suivante

$$Q = h_c \cdot (T_S - T_E)$$

où $h_c$ est le coefficient de convection, $T_S$ la température de surface et $T_E$ la température ambiante et

   - **en ce que** le coefficient de convection $h_C$ se trouve dans une plage de 3,0 à 3,6 $W/m^2$, de préférence à 3,3 $W/m^2$, lorsqu'une convection exclusivement libre se produit.

**8.** Procédé selon la revendication 7,
   **caractérisé en ce que**
   l'on fait varier le coefficient de convection $h_c$ correspondant aux conditions d'écoulement survenant à la surface de l'organisme pour le corps type (10) et/ou pour l'environnement (14).

**9.** Procédé selon l'une quelconque des revendications 1 à 8, **caractérisé en ce que**

   - lors de la détermination du transfert thermique global une production thermique post mortem de l'organisme est prise en compte, la production thermique post mortem interne initiale de l'organisme correspondant de préférence au niveau du métabolisme basal de l'organisme immédiatement avant le décès de celui-ci, et
   - **en ce que** la production thermique post mortem décroît de façon linéaire ou exponentielle à partir de l'heure théorique du décès.

**10.** Procédé selon la revendication 9,
   **caractérisé en ce que** la production thermique interne post mortem est déterminée selon la fonction

$$P(t) = P_o \cdot \exp(-\alpha \cdot t)$$

où P désigne le rendement thermique interne post mortem, $P_o$ désigne la valeur initiale du rendement interne et $\alpha$ désigne le taux de diminution du rendement interne et le taux de diminution $\alpha$ présente de préférence une valeur située dans une plage de 0,00002 $S^{-1}$ à 0,00020 $S^{-1}$ et notamment une valeur équivalente à 0,000077 $S^{-1}$.

**11.** Procédé selon la revendication 9 ou 10, **caractérisé en ce que**,

   - la production thermique interne est répartie indifféremment en pourcentage sur plusieurs groupes d'éléments volumiques (18-42) formant des compartiments corporels (16) de l'organisme et
   - lorsque l'heure du décès d'un être humain est déterminée à l'aide du procédé, on attribue 55 à 65 % du métabolisme basal de l'être humain au tronc, 16 à 20 % du métabolisme basal à la musculature, 15 à 19 % du métabolisme basal au cerveau et 4 à 6 % du métabolisme basal aux os et aux tissus conjonctifs.

**12.** Procédé selon l'une quelconque des revendications précédentes,
   **caractérisé en ce que**
   lors de la détermination du transfert thermique global les températures ambiantes se modifiant au fil du temps sont également prises en considération.

**13.** Procédé selon l'une quelconque des revendications précédentes, **caractérisé en ce qu'**un rayonnement externe est pris en considération.

**14.** Procédé selon l'une quelconque des revendications précédentes,
   **caractérisé en ce que**
   diverses phases de positionnement successives sont recensées avec des conditions ambiantes différentes, le champ de températures calculé étant utilisé à la fin d'une phase est utilisé comme champ de températures initial pour la phase suivante.

**15.** Procédé selon l'une quelconque des revendications précédentes,
   **caractérisé en ce que**
   le corps type (10) est généré dans sa forme, dans ses proportions, dans sa posture et/ou dans son maintien d'une manière correspondant au moins approximativement à la forme, aux proportions, à la posture et/ou au maintien de l'organisme à expertiser.

**16.** Procédé selon l'une quelconque des revendications précédentes, **caractérisé en ce que**

-   le procédé est utilisé pour déterminer l'heure du décès d'un être humain,
-   la température rectale ou les températures à d'autres points de mesure de l'être humain décédé sont mesurées en vue de les comparer avec la modification de température théorique (46 - 56, 60, 62) du corps type (10) et
-   le corps type (10) généré sous la forme d'un être humain présente un élément volumique, qui est utilisé conformément à sa position dans le corps type (10) comme point de mesure de comparaison (44) pour la température rectale ou les températures à d'autres points de mesure, la modification de température théorique (46 - 56, 60, 62) au niveau de cet élément volumique étant comparée avec la température rectale mesurée pour permettre la détermination de l'heure du décès.

**17.** Procédé selon l'une quelconque des revendications précédentes, **caractérisé en ce que**

-   l'équation différentielle partielle du rendement thermique décrivant le transfert thermique global du corps type (10) est résolue à l'aide d'un procédé numérique, dans lequel les propriétés thermodynamiques sont utilisées comme paramètres dans l'équation différentielle du rendement thermique et
-   **en ce qu'**on prédétermine le niveau de température initial comme condition initiale et une température ambiante en tant que condition marginale pour la résolution numérique de l'équation différentielle du rendement thermique partielle.

Fig.1

Haut

Fett

Muskel

Knochen

Hirn

Gesicht

Hals

Mediastinum

Lunge

Bauch

MDT

Becken

Nierenlager

Stahl

Fig.2

18

Fig. 3

Fig.4

Fig.5

24

Fig.6

26

28

30

Fig.7

30

32

34

Fig.8

Fig.9

Fig.10

Fig.11

Inc : 26
Time : 7.575e+003

3.640e+001

3.423e+001

3.207e+001

2.990e+001

2.774e+001

2.557e+001

2.340e+001

2.124e+001

1.907e+001

1.691e+001

1.474e+001

14

10

12

Fig.12

Fig.13

Inc :  33
Time : 1.152e+005

1.759e+001

1.713e+001

1.668e+001

1.622e+001

1.577e+001

1.531e+001

1.486e+001

1.440e+001

1.395e+001

1.349e+001

1.304e+001

Fig.14

Fig.15

EP 1 221 297 B1

Fig.16

34

Fig.17

EP 1 221 297 B1

Fig.18

Fig.19

EP 1 221 297 B1

Fig.20

Fig.21

Fig.22

Fig.23

Inc : 29
Time : 2.557e+004

58

3.720e+001
3.478e+001
3.236e+001
2.994e+001
2.752e+001
2.510e+001
2.268e+001
2.026e+001
1.784e+001
1.542e+001
1.300e+001

14

10

12

42

EP 1 221 297 B1

Fig. 24

Fig.25

Fig. 26